# EUROPÄISCHE PATENTSCHRIFT

(11) **EP 0 772 616 B1**
(45) Veröffentlichungstag und Bekanntmachung des Hinweises auf die Patenterteilung: **15.12.1999**
(21) Anmeldenummer: 95926976.2
(22) Anmeldetag: 24.07.1995
(51) Int. Cl.: C07F 15/00, C12Q 1/68, G01N 33/58, C07F 19/00

(54) **HYDROPHILE METALLKOMPLEXE**
HYDROPHILIC METAL COMPLEXES
COMPLEXES METALLIQUES HYDROPHILES

(30) Priorität: 25.07.1994 DE 4426276; 31.08.1994 DE 4430998; 04.11.1994 DE 4439346
(43) Veröffentlichungstag der Anmeldung: 14.05.1997
(73) Patentinhaber: Roche Diagnostics GmbH, 68305 Mannheim (DE)
(72) Erfinder: HERRMANN, Rupert, D-82362 Weilheim (DE); JOSEL, Hans-Peter, D-82362 Weilheim (DE); PAPPERT, Gunter, D-82319 Starnberg (DE); VÖGTLE, Fritz, D-53347 Alfter-Impekoven (DE); FROMMBERGER, Bruno, D-53123 Bonn (DE); ISSBERNER, Jörg, D-53913 Swisttal (DE)
(74) Vertreter: Weiss, Wolfgang, Dipl.-Chem. Dr.
(86) Internationale Anmeldenummer: EP9502923
(87) Internationale Veröffentlichungsnummer: WO9603410

(56) Entgegenhaltungen:
- WO-A-86/02734
- WO-A-87/06706
- WO-A-92/14139
- JOURNAL OF THE AMERICAN CHEMICAL SOCIETY, Bd.116, Nr.8, 20. April 1994 Seiten 3399 - 3404 SEILER, M. ET AL. 'PHOTOINDUCED ELECTRON TRANSFER IN SUPRAMOLECULAR ASSEMBLIES COMPOSED OF DIALKOXYBENZENE-TETHERED RUTHENIUM(II) TRISBIPYRIDINE AND BIPYRIDINIUM SALTS'
- CHEMICAL ABSTRACTS, vol. 98, no. 22, 30. Mai 1983, Columbus, Ohio, US; abstract no. 188896x, MARKOVITSI, D. ET AL. 'ANNELIDES. VI. PHOTOCHEMICAL PROPERTIES OF MICELLAR PHASES OF METAL ION COMPLEXES' Seite 587 ; & NOUV. J. CHIM., Bd.6, Nr.11, 1982 Seiten 531 - 537
- Chem. Eur. J. 3 (1997), 706-712
- H. R. Christen "Grundlagen der Organischen Chemie" (1982),Seiten 31-33

## Beschreibung

Die der vorliegenden Erfindung zugrundeliegende Aufgabe bestand somit darin, die Nachteile des Standes der Technik mindestens teilweise zu beseitigen.

Überraschenderweise wurde festgestellt, daß die Einführung von C₂-C₃-Alkylenoxy-, C₂-C₃-Alkylenthio- oder/und C₂-C₃-Alkylenamino-Einheiten und insbesondere von Ethylenglycol- oder/und Propylenglycol-Einheiten in lumineszierende Metallkomplexe die Adsorption von Konjugaten dieser Komplexe mit einer immunologisch reaktiven Substanz verringert und damit auch die Stabilität und Wiederfindnung der Konjugate in Immunoassays verbessert. Überdies kann eine erhöhte Quantenausbeute erzielt werden.

Weiterhin wurde festgestellt, daß die Eigenschaften von Metallkomplexen auch durch Einführung von Polyhydroxy-Einheiten verbessert werden können. Diese Polyhydroxy-Einheiten können zu dendrimerartigen Strukturen mit mehreren Generationen ausgebaut werden. Außerdem kann durch Einbau von Polyamin-Strukturen der für Elektrochemilumineszenzmessungen benötigte Elektronendonor direkt in die Ligandensphäre des Komplexes integriert werden.

Eine weitere erfindungsgemäße Verbesserung betrifft Metallkomplexe in Form eines Käfigs oder Halbkäfigs, in dem die Liganden einfach oder mehrfach, vorzugsweise über hydrophile Spacer, miteinander verknüpft sind. Auch dies führt zu einer wesentlichen Verbesserung der Photostabilität und zu einer Verringerung des Sauerstoff-Quenchings.

Ein Gegenstand der vorliegenden Erfindung ist somit ein Metallkomplex mit der allgemeinen Formel (I):

[M(L₁L₂L₃)]ₙ - Xₘ A (I)

worin M ein zwei- oder dreiwertiges Metallkation ausgewählt aus Seltenerde- oder Übergangmetallionen ist, L₁, L₂ und L₃ Seiler et al. (J. Am. Chem. Soc., 116 (1994), 3399-3404) beschreiben einen photoindizierten Elektronentransfer in supramolekularen Anordnungen, die aus Dialkoxybenzol-substituierten Ruthenium (II)-Trisbipyridin-Komplexen und Bipyridinium-Salzen bestehen. Markovitsi (Nouv. J. Chim. 6 (1982), 531-537) beschreiben die Synthese von mit Oxyalkyleneinheiten substituierten Ruthenium (II)-Trisbipyridin-Komplexen. Keines der beiden Dokument enthält Hinweise auf die Verwendung derartiger Komplexe zur Kopplung an biolgische Substanzen und als Markierungsgruppe in diagnostischen Nachweisverfahren.

Die der vorliegenden Erfindung zugrundeliegende Aufgabe bestand somit darin, die Nachteile des Standes der Technik mindestens teilweise zu beseitigen.

Überraschenderweise wurde festgestellt, daß die Einführung von C₂-C₃-Alkylenoxy-, C₂-C₃-Alkylenthio- oder/und C₂-C₃-Alkylenamino-Einheiten und insbesondere von Ethylenglycol- oder/und Propylenglycol-Einheiten in lumineszierende Metallkomplexe die Adsorption von Konjugaten dieser Komplexe mit einer immunologisch reaktiven Substanz verringert und damit auch die Stabilität und Wiederfindnung der Konjugate in Immunoassays verbessert. Überdies kann eine erhöhte Quantenausbeute erzielt werden.

Weiterhin wurde festgestellt, daß die Eigenschaften von Metallkomplexen auch durch Einführung von Polyhydroxy-Einheiten verbessert werden können. Diese Polyhydroxy-Einheiten können zu dendrimerartigen Strukturen mit mehreren Generationen ausgebaut werden. Außerdem kann durch Einbau von Polyamin-Strukturen der für Elektrochemilumineszenzmessungen benötigte Elektronendonor direkt in die Ligandensphäre des Komplexes integriert werden.

Eine weitere erfindungsgemäße Verbesserung betrifft Metallkomplexe in Form eines Käfigs oder Halbkäfigs, in dem die Liganden einfach oder mehrfach, vorzugsweise über hydrophile Spacer, miteinander verknüpft sind. Auch dies führt zu einer wesentlichen Verbesserung der Photostabilität und zu einer Verringerung des Sauerstoff-Quenchings.

Ein Gegenstand der vorliegenden Erfindung ist somit ein Metallkomplex mit der allgemeinen Formel (I):

[M(L₁L₂L₃)]ₙ - Xₘ A (I)

worin M ein zwei- oder dreiwertiges Metallkation ausgewählt aus Seltenerde- oder Übergangmetallionen ist, L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind, X eine reaktive oder aktivierbare funktionelle Gruppe ist, die an mindestens einen der Liganden L₁, L₂, L₃ kovalent gebunden ist, n eine ganze Zahl von 1 bis 10 ist, m eine ganze Zahl von 1 bis 6 und vorzugsweise von 1 bis 3 ist und A eine oder mehrere zum Ladungsausgleich erforderliche, negativ geladene Gruppen bedeutet, wobei der Komplex mindestens eine hydrophile Gruppe ausgewählt aus C₂-C₃-Alkylenoxy-Einheiten, C₂-C₃-Alkylenthio-Einheiten, C₂-C₃-Alkylenamino-Einheiten und Polyhydroxy-Einheiten enthält, mit der Maßgabe, daß die Gruppe X keine Hydroxy-, Methoxy-, 4-Methoxyphenoxy-, Dioxolan- oder CO₂CH₃-Gruppe ist, wobei die hydrophilen Gruppen Bestandteile von Substituenten der Liganden des Metallkomplexes sind und in einem Linker zwischen einem der Liganden und der Gruppe X oder/und in einfachen, nicht die Gruppe X enthaltenden Substituenten vorliegen.

Der Metallkomplex ist vorzugsweise ein lumineszierender Metallkomplex, d.h. ein Metallkomplex, der eine nachweisbare Lumineszenzreaktion erzeugen kann. Der Nachweis dieser Lumineszenzreaktion kann beispielsweise durch Fluoreszenz- oder durch Elektrochemilumineszenzmessung erfolgen. Das Metallkation in diesem Komplex ist beispielsweise ein Übergangsmetall oder ein Seltenerdenmetall. Vorzugsweise ist das Metall Ruthenium, Osmium, Rhenium, Iridium, Rhodium, Platin, Indium, Palladium, Molybdän, Techneticum, Kupfer, Chrom oder Wolfram. Besonders bevorzugt sind Ruthenium, Iridium, Rhenium, Chrom und Osmium. Am meisten bevorzugt ist Ruthenium.

Die Liganden L₁, L₂ und L₃ sind Liganden mit mindestens zwei stickstoffhaltigen Heterocyclen. Bevorzugt sind aromatische Heterocyclen wie z.B. Bipyridyl, Bipyrazyl, Terpyridyl und Phenanthrolyl. Besonders bevorzugt werden die Liganden L₁, L₂ und L₃ aus Bipyridin- und Phenanthrolin-Ringsystemen ausgewählt.

Die reaktive oder aktivierbare funktionelle Gruppe X des Komplexes ist eine reaktive Gruppe, die mit einer immunologisch aktiven Substanz gekoppelt werden kann, oder eine aktivierbare Gruppe, die auf einfache Weise in eine solche reaktive Gruppe überführt werden kann. Vorzugsweise ist die Gruppe X eine aktivierte Carbonsäuregruppe wie etwa ein Carbonsäurehalogenid, ein Carbonsäureanhydrid oder ein Aktivester, z.B. ein N-Hydroxysuccinimid-, ein p-Nitrophenyl-Pentafluorphenyl-, Imidazolyl- oder N-Hydroxybenzotriazolylester, ein Maleimid, ein Amin, eine Carbonsäure, ein Thiol, ein Halogenid oder eine photoaktivierbare Gruppe.

Weiterhin enthält der Komplex eine oder mehrere zum Ladungsausgleich erforderliche, negativ geladene Gruppen A. Beispiele für geeignete negativ geladene Gruppen sind Halogenide, OH⁻, Carbonat, Alkylcarboxylat, z.B. Trifluoracetat, Sulfat, Hexafluorophosphat- und Tetrafluoroborat-Gruppen. Hexafluorophosphat-, Trifluoracetat und Tetrafluoroborat-Gruppen sind besonders bevorzugt.

Der erfindungsgemäße Metallkomplex unterscheidet sich von den aus dem Stand der Technik bekannten Metallkomplexen dadurch, daß er mindestens eine hydrophile Gruppe ausgewählt aus C₂-C₃-Alkylenoxy-Einheiten, C₂-C₃-Alkylenthio-Einheiten, C₂-C₃-Alkylenamino-Einheiten und Polyhydroxy-Einheiten enthält.

Die Polyhydroxy-Einheiten werden vorzugsweise aus Gruppen der Formeln (IIa) oder (IIb) ausgewählt:

-NR-W (IIa) -O-W (IIb)

worin W einen organischen Rest mit mindestens zwei Hydroxygruppen und R Wasserstoff oder C₁-C₅-Alkyl, vorzugsweise Wasserstoff oder C₁-C₃-Alkyl bedeutet. Der organische Rest W enthält vorzugsweise 2 bis 6 und besonders bevorzugt 2 bis 4 Hydroxygruppen. Weiterhin sollte W günstigerweise 2 bis 10 und insbesondere 3-6 Kohlenstoffatome enthalten. Spezifische Beispiele für geeignete Polyhydroxy-Einheiten sind Reste von Polyalkoholen wie etwa Glycerin oder Aminopolyalkoholen. Ein bevorzugter Aminopolyalkohol ist Tris (2-Amino-2-(hydroxymethyl)-1,3-propantriol). In diesem Fall weist die Polyhydroxy-Einheit die Formel NR-C(CH₂OH)₃ auf. Die Polyalkohole bzw. Aminopolyalkohole sind an dem Metallkomplex vorzugsweise in Form von Estern bzw. Amiden gekoppelt.

Die C₂-C₃-Alkylenoxy-, C₂-C₃-Alkylenthio- und C₂-C₃-Alkylenamino-Einheiten des erfindungsgemäßen Metallkomplexes sind vorzugsweise C₂-Einheiten und insbesondere Ethylenoxy-Einheiten. Der Komplex enthält pro Metallkation vorzugsweise 1 bis 30 und besonders bevorzugt 2 bis 20 C₂-C₃-Alkylen-oxy-, C₂-C₃-Alkylenthio- bzw. C₂-C₃-Alkylenamino-Einheiten. Diese Einheiten sind Bestandteile von Substituenten der heterocyclischen Liganden des Metallkomplexes. Sie können in einem Linker zwischen einem der Liganden und der reaktiven oder aktivierbaren funktionellen Gruppe X oder/und in einfachen Substituenten vorliegen. Die Alkylenoxy-, Alkylenthio-bzw. Alkylenamino-Einheiten können auch über einen Brückenkopf miteinander verknüpft sein, der gegebenenfalls eine funktionelle Gruppe X tragen kann. Andererseits können über den Brückenkopf auch mehrere Komplex-Einheiten miteinander verknüpft sein. Beispiele für bevorzugte Ausführungsformen der erfindungsgemäßen Metallkomplexe sind im folgenden angegeben.

In einer ersten bevorzugten Ausführungsform der vorliegenden Erfindung besitzt der erfindungsgemäße Metallkomplex die allgemeine Formel (III): 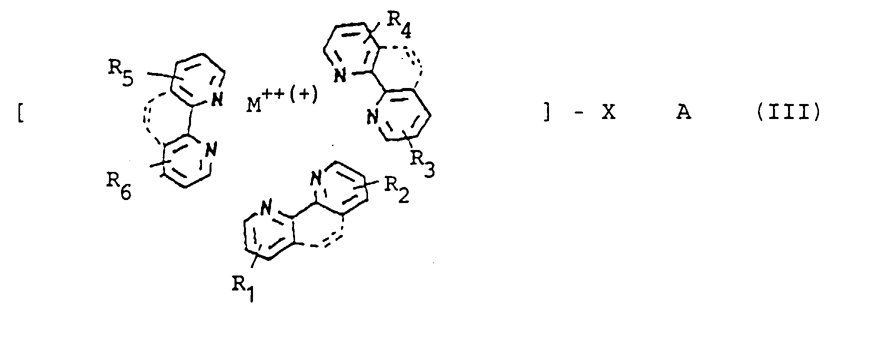 worin M, X und A wie vorstehend definiert sind, R₁, R₂, R₃, R₄, R₅ und R₆ gleich oder verschieden sind und jeweils einen oder mehrere Substituenten bedeuten, unter der Voraussetzung, daß X über einen der Substituenten R₁, R₂, R₃, R₄, R₅ oder R₆ an einen der Liganden gebunden ist und daß mindestens einer der Substituenten R₁, R₂, R₃, R₄, R₅ oder R₆ mindestens eine hydrophile Gruppe, ausgewählt aus C₂-C₃-Alkylenoxy-, C₂-C₃-Alkylenthio- und C₂-C₃-Alkylenamino-Einheiten enthält.

Die Liganden des Komplexes sind je nach Anwesenheit bzw. Abwesenheit der durch gebrochene Linien bezeichneten Gruppen, gegebenenfalls substituierte Phenanthrolin- bzw. Bipyridinsysteme.

Die Substituenten R₁, R₂, R₃, R₄, R₅ und R₆ an den Liganden sind - sofern sie keine hydrophile Gruppe enthalten - vorzugsweise Wasserstoff, C₁-C₅-Alkyl, insbesondere C₁-C₃-Alkyl. Insgesamt enthalten die hydrophilen Gruppen vorzugsweise 1 bis 30 und besonders bevorzugt 2 bis 20 Alkylenoxy-, Alkylenthio- oder/und Alkylenamino-Einheiten, insbesondere Ethylenoxy-Einheiten.

Die hydrophile Gruppe kann Bestandteil eines Linkers zwischen der funktionellen, kopplungsfähigen Gruppe X und einem der Liganden sein. In diesem Fall besitzen die Metallkomplexe vorzugsweise die allgemeine Formel (IIIa) : 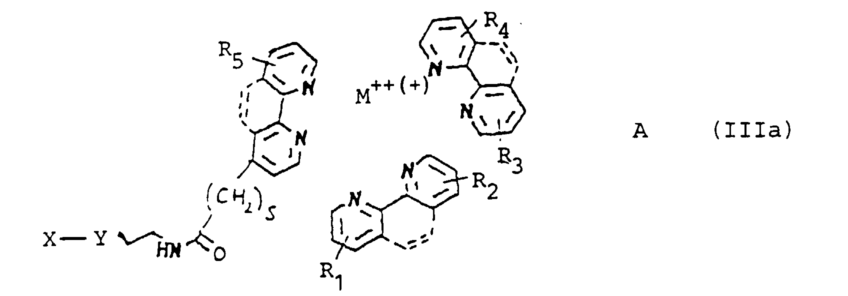 worin M, X und A wie vorstehend definiert sind, R₁, R₂, R₃, R₄ und R₅ wie vorstehend definiert sind, s eine ganze Zahl von 0 bis 6 vorzugsweise von 1 bis 4 ist und Y eine hydrophile Linkergruppe mit 1 bis 10, vorzugsweise mit 2 bis 6 hydrophile Einheiten bedeutet, ausgewählt aus C₂-C₃-Alkylenoxy-, C₂-C₃-Alkylenthio und C₂-C₃-Alkylenamino-Einheiten, insbesondere Ethylenoxy-Einheiten.

Die funktionelle Gruppe X muß jedoch nicht über einen hydrophilen Linker mit dem Liganden verknüpft sein. In diesem Fall besitzt der erfindungsgemäße Metallkomplex vorzugsweise die allgemeine Formel (IIIb) : 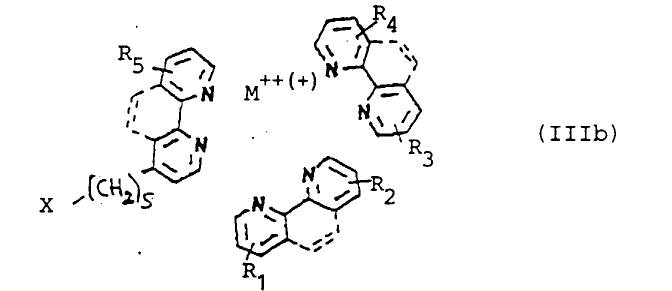 worin M, X und A wie vorstehend definiert sind, R₁, R₂, R₃, R₄ und R₅ wie vorstehend definiert sind, unter der Voraussetzung, daß R₁, R₂, R₃, R₄ oder/und R₅ eine hydrophile Substituentengruppe enthält, die jeweils 1 bis 10 vorzugsweise 2 bis 6 C₂-C₃-Alkylenoxy-, C₂-C₃-Alkylenthio oder/und C₂-C₃-Alkylenamino-Einheiten, insbesondere Ethylenoxy-Einheiten umfaßt.

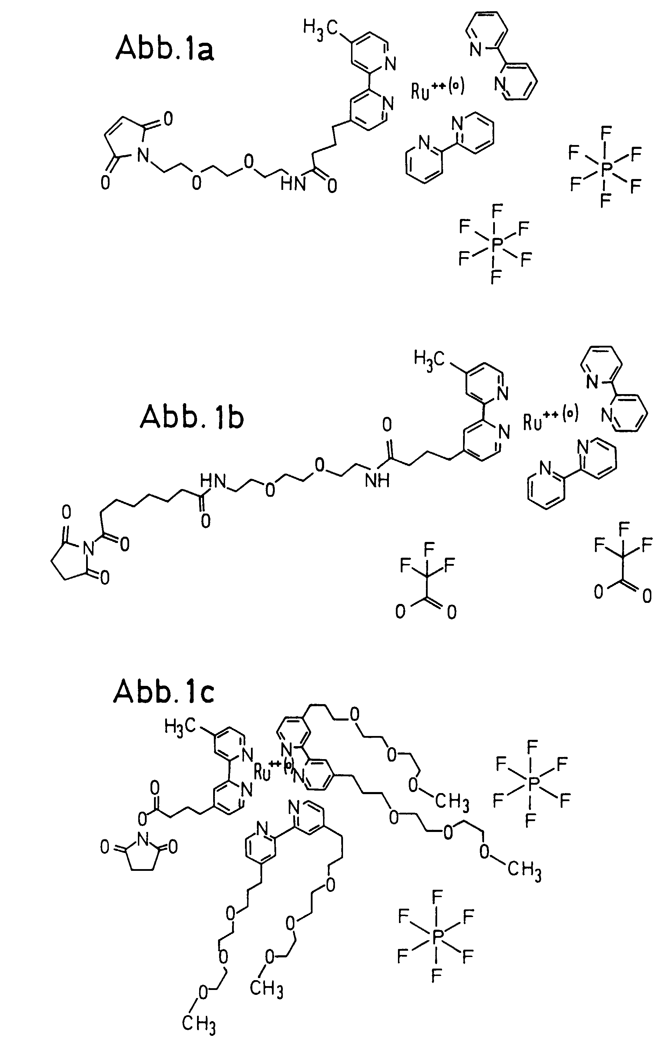

Ein Beispiel für eine Verbindung der Formel (IIIa) ist in Abb. 1a und 1b gezeigt. Diese Verbindungen enthalten die Hydrophilie nur im Linker zwischen der Gruppe X - einem Maleimid (Abb. 1a) bzw. einem N-Hydroxysuccinimidester (Abb. 1b) - und einem Liganden. Ebenso können aber auch die anderen Liganden hydrophile Substituenten aufweisen. Ein Beispiel für eine Verbindung der Formel (IIIb) ist in Abb. 1b gezeigt. Hier ist die Gruppe X ein N-Hydroxysuccinimidester.

Die Liganden des erfindungsgemäßen Metallkomplexes können auch miteinander verknüpft sein, so daß der Metallkomplex in Form eines Halbkäfigs bzw. Käfigs vorliegt. Eine bevorzugte Ausführungsform eines erfindungsgemäßen Metallkomplexes in Form eines Halbkäfigs oder Käfigs besitzt die allgemeine Formel (IV) : 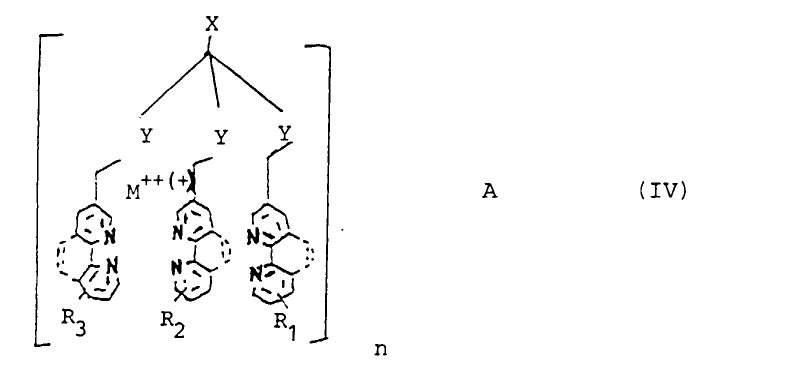 worin M, X, n und A wie vorstehend definiert sind, R₁, R₂ und R₃ gleich oder verschieden sind und jeweils einen oder mehrere Substituenten - wie vorstehend definiert - an dem Bipyridin- oder Phenanthrolin-Liganden bedeuten und Y jeweils eine hydrophile Linkergruppe bedeutet, die 1 bis 10 hydrophile Einheiten, ausgewählt aus C₂-C₃-Alkylenoxy-, C₂-C₃-Alkylenthio- und C₂-C₃-Alkylenamino-Einheiten, insbesondere Ethylenoxy-Einheiten umfaßt.

Wenn die Substituenten R₁, R₂ und R₃ in Formel (IV) und gegebenenfalls über hydrophile Linkergruppen kovalent miteinander verknüpft sind, dann besitzt der Komplex der Formel (IV) die Form eines Käfigs.

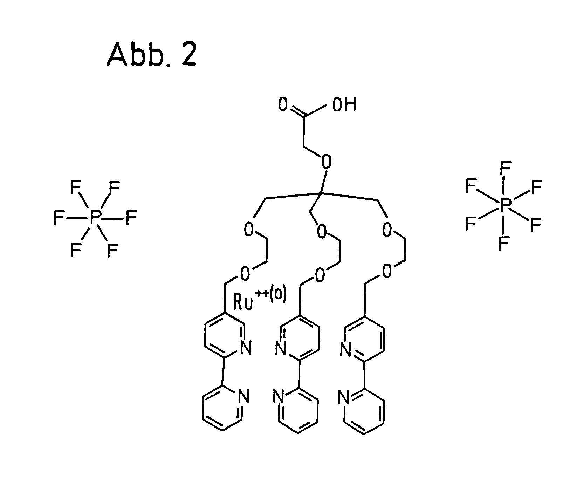

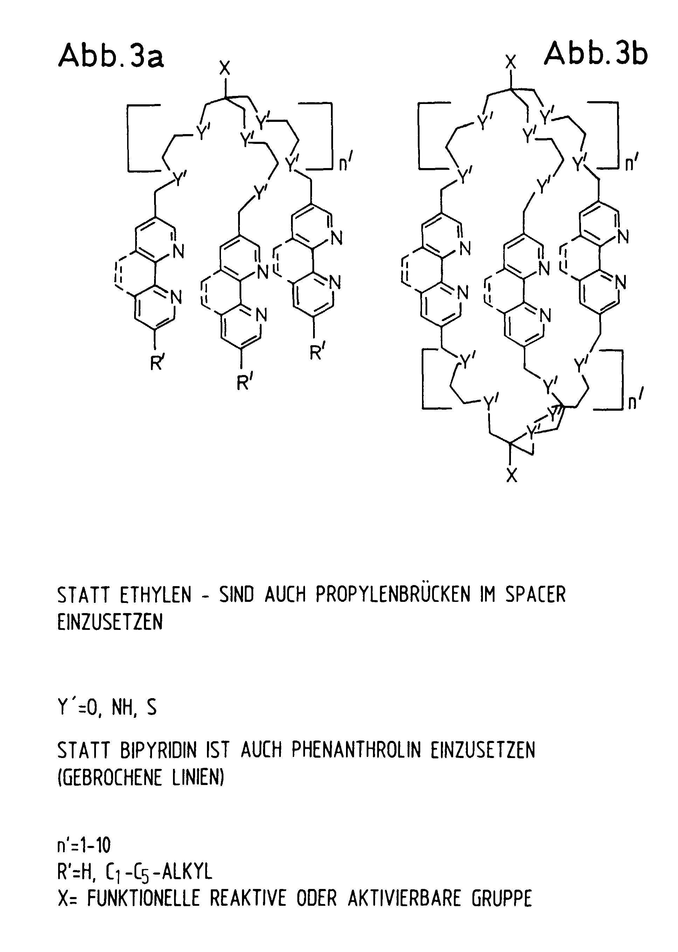

Beispiele für halbkäfigförmige Komplexe der Formel (IV) sind in Abb. 2 und 3a gezeigt. Ein Beispiel für einen käfigförmigen Komplex ist in Abb. 3b gezeigt. Die Gruppe X in Abb. 2 ist ein Carboxylrest. Das Metallkation und die Anionen sind in Abb. 3a und 3b nicht dargestellt.

Der Komplex der Formel (IV) kann nicht nur als Monomer, sondern als Oligomer aus vorzugsweise bis zu 5 einzelnen Metallkomplexen vorliegen. Hierzu kann die funktionelle kopplungsfähige Gruppe X beispielsweise ein Substituent an einem aromatischen Kern, z.B. einem Phenylkern sein, wobei zwei oder mehr der restlichen Substituentenpositionen des aromatischen Kerns durch einen halbkäfig- bzw. käfigförmigen Metallkomplex substituiert sein können.

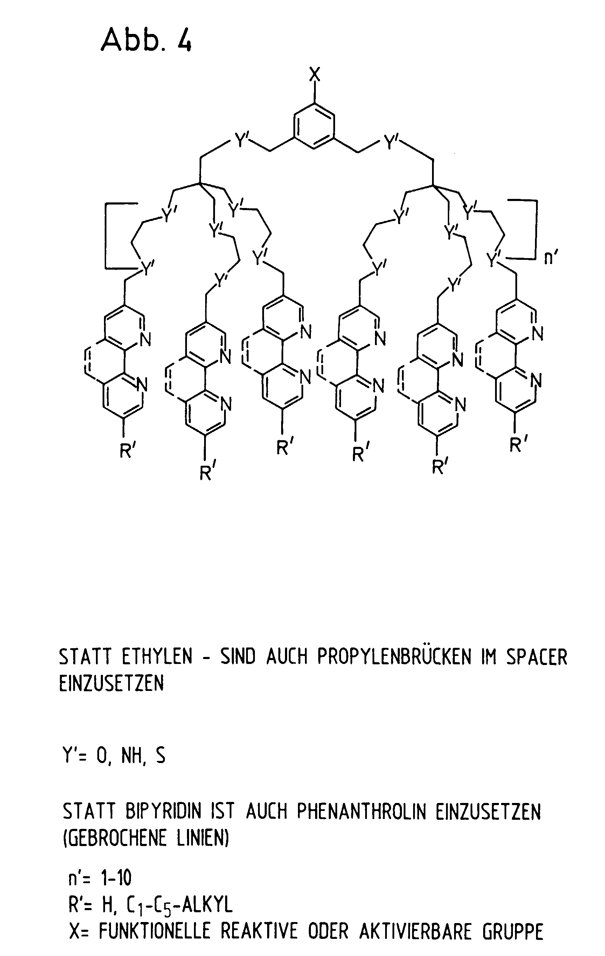

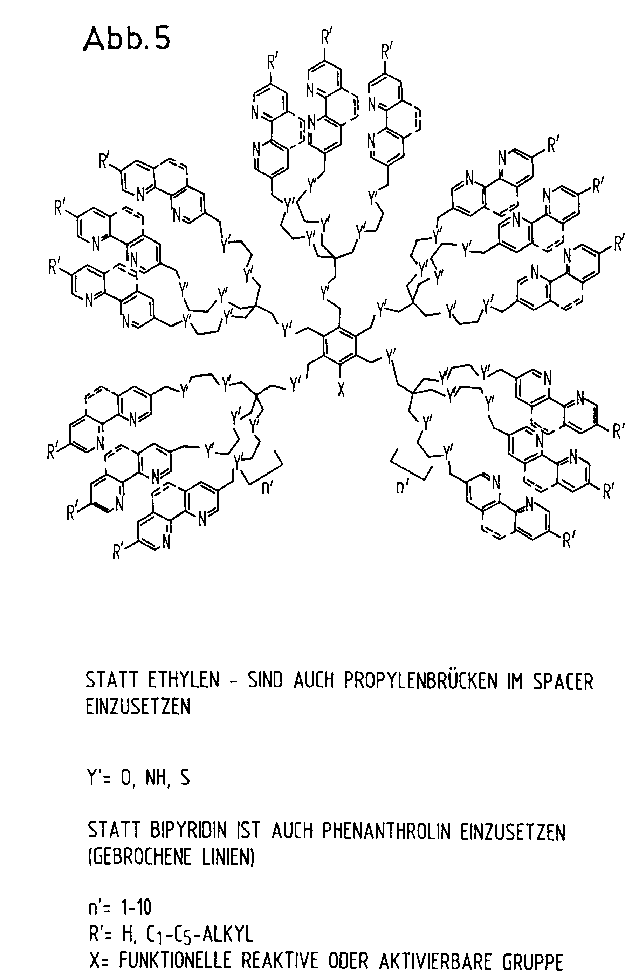

Beispiele für oligomere Metallkomplexe der Formel (IV) sind in Abb. 4 und 5 gezeigt. Die Metallionen und die Anionen sind in diesen Abbildungen nicht dargestellt.

In einer weiteren bevorzugten Ausführungsform der vorliegenden Erfindung ist der Metallkomplex mit Polyhydroxy-Einheiten substituiert und besitzt die allgemeine Formel (V) : 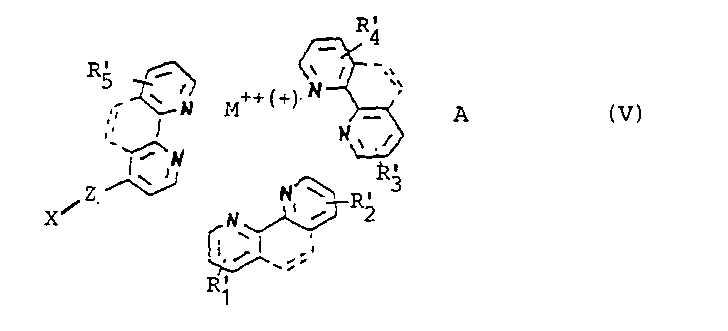 worin M, X und A wie vorstehend definiert sind, Z eine Linkergruppe bedeutet, R'₁, R'₂, R'₃, R'₄ und R'₅ gleich oder verschieden sind und jeweils einen oder mehrere Substituenten, z.B. Wasserstoff oder C₁-C₅-, insbesondere C₁-C₃-Alkyl, bedeuten und s eine ganze Zahl von 0 bis 6, vorzugsweise von 1 bis 4 ist, unter der Voraussetzung, daß R'₁, R'₂, R'₃ oder/und R'₄ eine hydrophile Substituentengruppe enthält, die eine Polyhydroxy-Einheit umfaßt.

Der Ligand X des Metallkomplexes (V) kann mit dem Liganden über einen hydrophilen Linker, z.B. einen Linker gemäß Formel (IIIa), aber auch über einen Linker gemäß Formel (IIIb) gekoppelt sein. Der Substituent R'₅ ist vorzugsweise Wasserstoff oder eine C₁-C₅-, insbesondere eine C₁-C₃-Alkylgruppe.

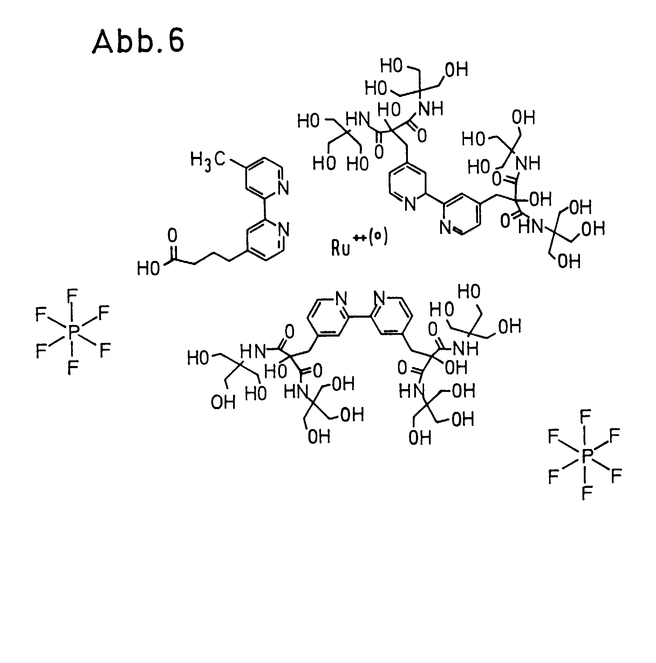

Ein Beispiel für eine Verbindung der Formel (V) ist in Abb.6 gezeigt. Die Gruppe X ist ein Carboxylrest.

Die OH-Gruppen der Polyhydroxy-Einheiten von Metallkomplexen der allgemeinen Formel (V) sind gegebenenfalls durch hydrophile Gruppen substituiert, z.B. durch C₂-C₃-Alkylenoxy; C₂-C₃-Alkylenthio- oder/und C₂-C₃-Alkylenamino-Einheiten.

In einer spezifischen Ausführungsform der vorliegenden Erfindung sind die hydrophilen Substituentengruppen der OH-Gruppen der Polyhydroxy-Einheiten Dendrimere der allgemeinen Formel (VIa) oder (VIb):

-A₁-NR-W₁ (A₂-NH-W₂)_{n'} (VIa)

-A₁-O-W₁(A₂-O-W₂)_{n'} (VIb)

worin
- A₁ und A₂ gleich oder verschieden sind und Linkergruppen bedeuten,
- W₁ und W₂ gleich oder verschieden sind und einen organischen Rest mit mindestens 2 Hydroxygruppen bedeuten,
- R Wasserstoff oder C₁-C₅-Alkyl und vorzugsweise Wasserstoff oder C₁-C₃-Alkyl bedeutet und
- n' 0 ist oder der Zahl der Hydroxygruppen von W₁ entspricht.

Die Linkergruppen A₁ und A₂ sind vorzugsweise Gruppen der Formel (CH₂)ₘ'C (=O)-, worin m' 1 bis 5 und insbesondere 1 bis 3 ist.

Die Gruppen W₁ und W₂ sind vorzugsweise Polyhydroxy-Einheiten, die entsprechend den Gruppen der Formeln (IIa), (IIb) definiert sind. Wenn n' 0 ist, liegt ein Dendrimer der ersten Generation vor. Wenn n' der Zahl der Hydroxygruppen von W₁ entspricht, liegt ein Dendrimer der zweiten Generation vor. Die Hydroxy-Endgruppen der Dendrimere können gegebenenfalls substituiert sein, z.B. durch eine Linkergruppe mit der Formel A₃-R', wobei A₃ wie die Linkergruppen A₁ und A₂ definiert ist, und R' C₁-C₅, vorzugsweise C₁-C₃-Alkyl bedeutet.

Die Herstellung der erfindungsgemäßen Metallkomplexe erfolgt durch Reaktion eines Metallsalzes, z.B. eines Metallhalogenids mit den entsprechenden Liganden und ggf. anschließenden Austausch des Halogenidions durch Hexafluorophosphat- oder Tetrafluoroborat-Anionen. Derartige Verfahren sind im Stand der Technik, z.B. in EP-B-0 178 450 und EP-B-0 255 534 beschrieben. Auf diese Offenbarung wird hiermit Bezug genommen.

Die Herstellung hydrophiler N-heterocyclischer Liganden kann auf einfache Weise durch Substitution am aromatischen Liganden, z.B. über ein Tosylat erfolgen. Auf entsprechende Weise kann auch eine Kopplung des hydrophilen Linkers, der die funktionelle Gruppe X trägt, erfolgen.

Die Herstellung von Metallkomplexen der Formel (IV) mit Halbkäfig- oder Käfigstruktur kann beispielsweise erfolgen durch Anfügen von Alkylenoxy-, Alkylenthio- oder/und Alkylenamino-Einheiten an die Bipyridin- oder Phenanthrolin-Liganden und Knüpfung dieser Einheiten an einen Brückenkopf über Ether oder Amidbindung. Bei Verwendung von zwei Brückenköpfen können Käfigstrukturen erhalten werden. Bevorzugt ist die Knüpfung von 3 Liganden an einen trivalenten Brückenkopf, z.B. Tris. Der Komplex selbst wird durch Umsetzung mit Metallsalzen, wie zuvor beschrieben, hergestellt.

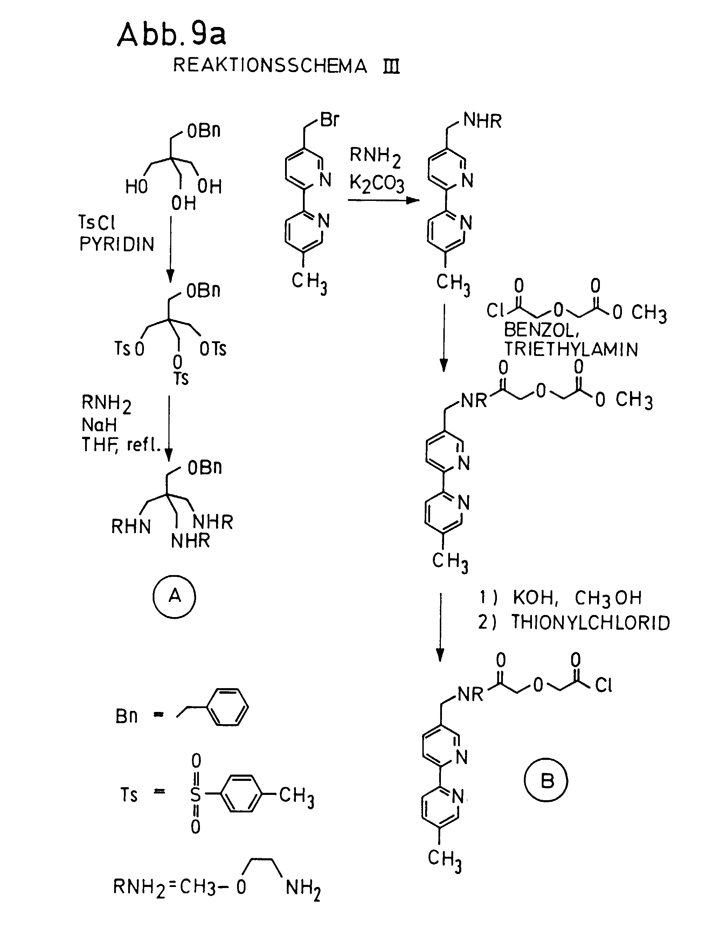

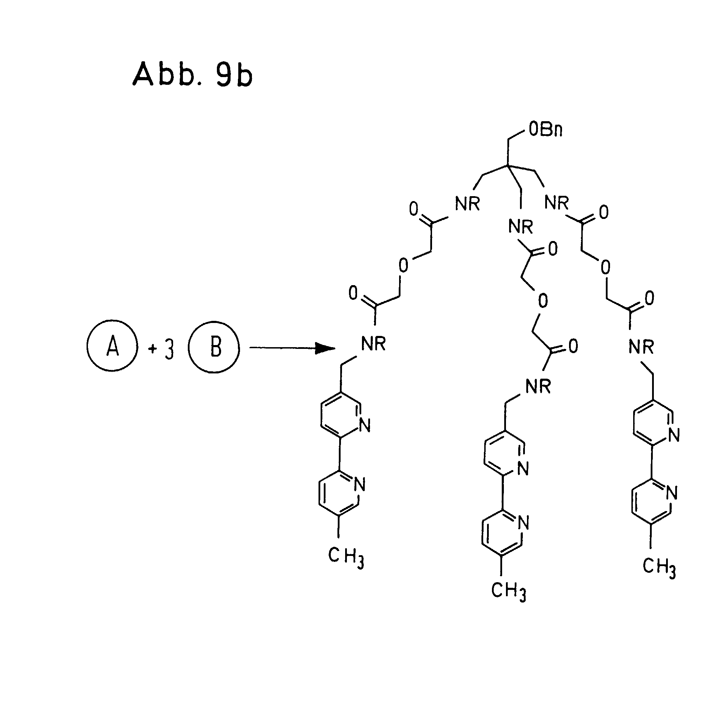

Die Herstellung von halbkäfig- und käfigförmigen Metallkomplexen kann gemäß Reaktionsschema III (Abb. 9a und 9b) erfolgen.

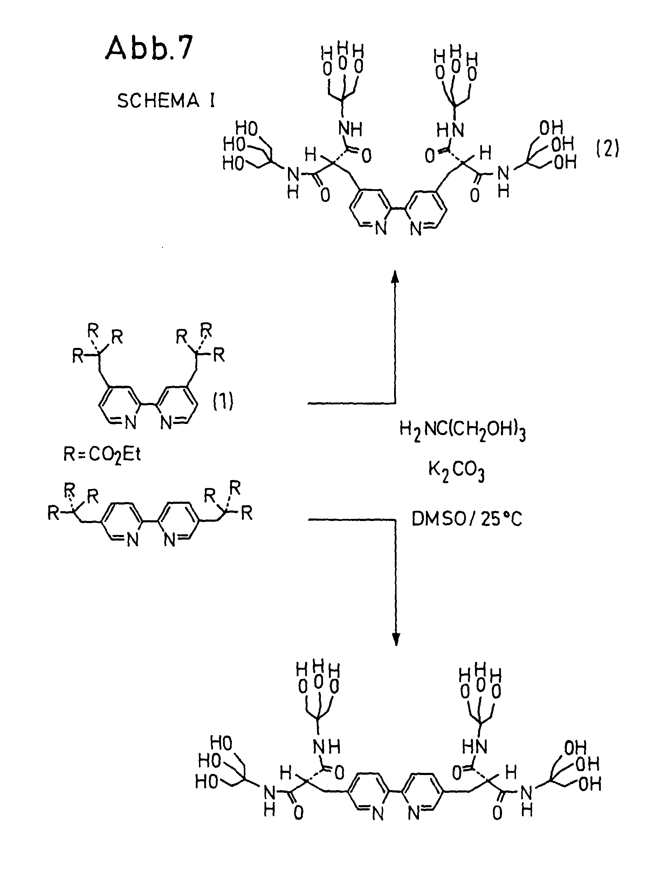

Die Herstellung von Metallkomplexen mit der allgemeinen Formel (V) erfolgt beispielsweise durch eine Reaktion gemäß Schema I (Abb. 7), indem ein entsprechend substituierter Ligand mit einem Aminopolyalkohol oder einem partiell geschützten Polyalkohol umgesetzt wird, wobei hydrophile Gruppen der Formeln (IIa) oder (IIb) an den Liganden angelagert werden.

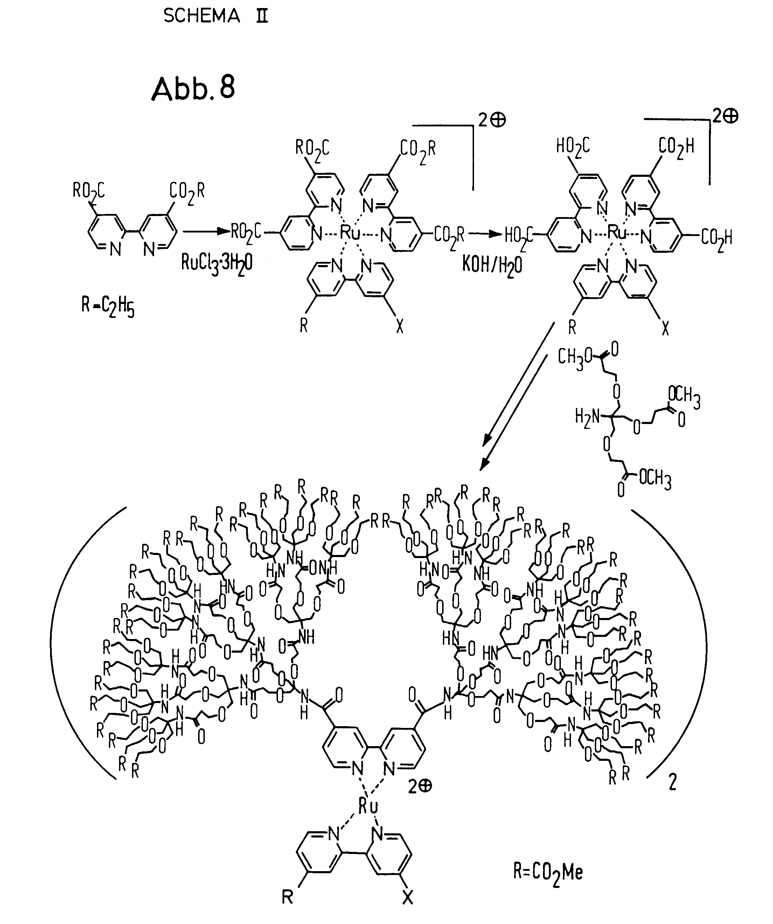

Die Herstellung von dendritischen Metallkomplexen kann entsprechend dem Reaktionsschema II (Abb. 8) erfolgen.

Ein weiterer Gegenstand der vorliegenden Erfindung ist ein Konjugat, umfassend eine-biologische Substanz, an die mindestens ein Metallkomplex mit der allgemeinen Formel (I) kovalent gekoppelt ist:

[M(L₁L₂L₃)]ₙ - Xₘ A (I)

worin
- M ein zwei- oder dreiwertiges Metallkation ausgewählt aus Seltenerde- oder Übergangsmetallionen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- X eine reaktive oder aktivierbare funktionelle Gruppe ist, die an mindestens einen der Liganden L₁, L₂, L₃ kovalent gebunden ist,
- n eine ganze Zahl von 1 bis 10 ist,
- m eine ganze Zahl von 1 bis 5 ist und
- A eine oder mehrere zum Ladungsausgleich erforderliche, negativ geladene Gruppe bedeutet,
wobei daß der Komplex mindestens eine hydrophile Gruppe ausgewählt aus C₂-C₃-Alkylenoxy-Einheiten, C₂-C₃-Alkylenthio-Einheiten, C₂-C₃-Alkylenamino-Einheiten oder/und Polyhydroxy-Einheiten enthält, wobei die hydrophilen Gruppen Bestandteile von Substituenten der Liganden des Metallkomplexes sind und in einem Linker zwischen einem der Liganden und der Gruppe X oder/und in einfachen, nicht die Gruppe X enthaltenden Substituenten vorliegen.

Beispiele für geeignete biologische Substanzen sind Zellen, Viren, subzelluläre Teilchen, Proteine, Lipoproteine, Glycoproteine, Peptide, Polypeptide, Nukleinsäuren, Oligosaccharide, Polysaccharide, Lipopolysaccharide, zelluläre Metaboliten, Haptene, Hormone, pharmakologische Wirkstoffe, Alkaloide, Steroide, Vitamine, Aminosäuren und Zucker.

Die Kopplung des Metallkomplexes mit der biologisch aktiven Substanz erfolgt über die reaktive oder aktivierbare funktionelle Gruppe des Metallkomplexes, die mit einer funktionellen Gruppe der biologischen Substanz kovalent kuppeln kann. Wenn die funktionelle Gruppe ein Aktivester ist, kann beispielweise eine Kopplung mit freien Aminogruppen der biologischen Substanz erfolgen. Wenn die funktionelle Gruppe ein Maleimidrest ist, kann eine Kopplung mit freien SH-Gruppen der biologischen Substanz erfolgen. Auf analoge Weise kann auch eine Aktivierung von funktionellen Gruppen der biologischen Substanz erfolgen, die anschließend beispielsweise mit einer freien Carbonsäure-, Amino- oder Thiol-funktion des Metallkomplexes reagieren können.

Bei einer besonders bevorzugten Ausführungsform der vorliegenden Erfindung werden die Metallkomplexe an ein Peptid gekoppelt, das vorzugsweise eine Länge von maximal 50 Aminosäuren und besonders bevorzugt von maximal 30 Aminosäuren aufweist. Die Herstellung dieser Metallkomplex-markierten Peptide erfolgt vorzugsweise dadurch, daß man ein Peptid mit der gewünschten Aminosäuresequenz an einer Festphase synthetisiert, wobei man a) nach der Synthese einen aktivierten Metallkomplex, vorzugsweise ein Metallkomplex-Aktivesterderivat an die N-terminale Aminogruppe des Peptids koppelt oder/und b) während der Synthese an mindestens einer Position des Peptids ein Aminosäurederivat einführt, das kovalent mit einem Metallkomplex gekoppelt ist. Die Kopplung des Metallkomplexes an die N-terminale Aminosäure des Peptids erfolgt vorzugsweise vor Abspaltung des Peptids von der Festphase und vor einer Abspaltung von Schutzgruppen an reaktiven Seitengruppen der zur Peptidsynthese verwendeten Aminosäurederivate.

Die Peptide enthalten vorzugsweise einen immunologisch reaktiven Epitopbereich und einen Spacerbereich, wobei mindesens eine Metallkomplex-Markierungsgruppe an den Spacerbereich gekoppelt wird. Der Spacerbereich weist vorzugsweise eine Länge von 1 bis 10 Aminosäuren auf und ist am Amino- oder/und Carboxyterminus des Peptids angeordnet.

Der Spacerbereich enthält vorzugsweise Aminosäuren, die Ladungen aufweisen oder/und Wasserstoffbrücken ausbilden können. Die Aminosäuren des Spacerbereichs werden vorzugsweise gebildet aus der Gruppe bestehend aus Glycin, β-Alanin, γ-Aminobuttersäure, ε-Aminocapronsäure, Lysin und Verbindungen der Strukturformel NH₂-[(CH₂)_{y}O]ₓ-CH₂-CH₂-COOH, worin y 2 oder 3 ist und x 1 bis 10 ist.

Die Epitopbereiche der Peptide stammen vorzugsweise aus pathogenen Organismen, z.B. Bakterien, Viren, und Protozoen, oder aus Autoimmun-Antigenen. Besonders bevorzugt stammt der Eptitopbereich aus viralen Antigenen, z.B. den Aminosäuresequenzen von HIVI, HIVII oder Hepatitis C-Virus (HCV).

Weitere bevorzugte Beispiele für biologische Substanzen sind Biotin, Nukleinsäuren, Antikörper oder Antikörperfragmente, Polypeptidantigene, d.h. immunologisch reaktive Polypeptide, oder Haptene, d.h. organische Moleküle mit einem Molekulargewicht von 150 bis 2000, insbesondere Moleküle mit einem Steroidgrundgerüst, wie etwa Cardenolide, Cardenolid-Glycoside (z.B. Digoxin, Digoxigenin), Steroid-Alkaloide, Sexualhormone (z.B. Progesteron), Glucocorticoide etc. Weitere Beispiele für Haptene sind Prostaglandine, Leuco-En-diine, Thromboxane, pharmakologische Wirkstoffe etc.

Noch ein weiterer Gegenstand der vorliegenden Erfindung ist die Verwendung der erfindungsgemäßen Metallkomplexe, bzw. der erfindungsgemäßen Konjugate in einem immunologischen Nachweisverfahren.

Dabei wird der Metallkomplex als Markierungsgruppe verwendet, mit deren Hilfe die qualitative oder/und quantitative Bestimmung eines Analyten in einer Probelösung möglich ist. Der Nachweis des Metallkomplexes erfolgt vorzugsweise durch Elektrochemilumineszenz, wobei lumineszierende Spezies elektrochemisch an der Oberfläche einer Elektrode erzeugt werden. Beispiele zur Durchführung von Lumineszenz-Assays mit Metallkomplexen des Standes der Technik finden sich in EP-A-0 580 979, WO 90/05301, WO 90/11511 und WO 92/14138. Auf die dort offenbarten Verfahren und Vorrichtungen für Lumineszenz-Assays wird hiermit Bezug genommen. Die Elektrochemilumineszenz-Assays werden in Gegenwart einer Festphase durchgeführt, die vorzugsweise aus Mikropartikeln, insbesondere aus magnetischen Mikropartikeln besteht, die mit einer reaktiven Beschichtung versehen sind, z.B. mit Streptavidin. Auf diese Weise können Immunkomplexe , die einen Metallkomplex als Markierungsgruppe enthalten, an die Festphase gebunden nachgewiesen werden.

Die Elektrochemilumineszenz-Messung wird vorzugsweise in Gegenwart eines Reduktionsmittels für den Metallkomplex durchgeführt, z.B. einem Amin. Bevorzugt sind aliphatische Amine, insbesondere primäre, sekundäre und tertiäre Alkylamine, deren Alkylgruppen jeweils 1 bis 3 Kohlenstoffatome aufweisen. Besonders bevorzugt ist Tripropylamin. Das Amin kann jedoch auch ein aromatisches Amin, wie Anilin oder ein heterocyclisches Amin sein. Das Reduktionsmittel kann bereits in der Ligandensphäre des Komplexes integriert sein. Derartige Systeme sind insbesondere zur Bestimmung von Analyten geeignet, die in hochkonzentrierter Form vorliegen.

Weiterhin kann ggf. Verstärker ein nichtionisches oberflächenaktives Mittel, z.B. ein ethoxyliertes Phenol vorhanden sein. Derartige Substanzen sind beispielsweise kommerziell unter den Bezeichnungen Triton X100 oder Triton N401 erhältlich.

Andererseits kann der Nachweis des lumineszierenden Metallkomplexes auch durch Fluoreszenz erfolgen, wobei das Metallchelat durch Bestrahlung mit einem Licht der geeigneten Wellenlänge angeregt und die daraus resultierende Fluoreszenzstrahlung gemessen wird. Beispiele zur Durchführung von Fluoreszenz-Assays finden sich in EP-A-0 178 450 und EP-A-0 255 534. Auf diese Offenbarung wird hiermit Bezug genommen.

Weiterhin wird die vorliegende Erfindung durch nachfolgende Beispiele und Abbildungen erläutert. Es zeigen:
- Abb. 1a: einen Metallkomplex der Formel (IIIa),
- Abb. 1b: einen Metallkomplex der Formel (IIIa),
- Abb. 1c: einen Metallkomplex der Formel (IIIb),
- Abb. 2: einen Metallkomplex der Formel (IV),
- Abb. 3a: einen Metallkomplex der Formel (IV),
- Abb. 3b: einen Metallkomplex der Formel (IV),
- Abb. 4: einen Metallkomplex der Formel (IV),
- Abb. 5: einen Metallkomplex der Formel (IV),
- Abb. 6: einen Metallkomplex der Formel (V),
- Abb. 7: ein Reaktionsschema zur Herstellung von Metallkomplexen der Formel (V),
- Abb. 8: ein weiteres Reaktionsschema zur Herstellung von Metallkomplexen der Formel (V),
- Abb. 9a und 9b: ein Reaktionschema zur Herstellung von Metallkomplexen der Formel (IV) und
- Abb. 10: ein Metallkomplex-Progesteron-Konjugat.

### Beispiel 1

### Herstellung eines hydrophilen Bipyridin-Liganden (4,4'-Bis (methoxy-ethoxy-ethoxy)-bipyridin)

50 ml einer Lösung von Lithiumdiisopropylamid in einem Gemisch aus Cyclohexan, Ethylbenzol und THF werden auf -78°C abgekühlt. Es werden 350 ml einer Lösung von 50 mmol Bipyridin in THF zugetropft. Man läßt zwei Stunden rühren und tropft dann eine Lösung von 100 mmol Methoxy-ethoxy-ethoxytosylat in THF hinzu. Nach einer Stunde bei -78°C läßt man das Reaktionsgemisch über Nacht bei Raumtemperatur stehen. Dann wird eine wäßrige Natriumchloridlösung zugegeben. Anschließend wird das THF mit einem Rotationsverdampfer entfernt und der Rückstand mit Essigester extrahiert.

Das Produkt wird chromatographisch über Kieselgel gereinigt. Eluens: Essigester-Methanol-Ammoniak 95/4/1 bzw. Amino-Kieselgel mit Essigester-Petrolether als Eluens.
- H-NMR(CDCl₃) :: 3,6 ppm (m.CH₂CH₂) = 16 H
7,12-8,5 ppm (bpy) = 6 H

### Beispiel 2

### Herstellung eines Bis(bisethylenglycol-bipyridin)-dichloro-Ruthenium-Komplexes

Rutheniumtrichlorid wird mit einem doppelten molaren Überschuß des in Beispiel 1 hergestellten Liganden und einem 7 bis 8 fachen Überschuß an Lithiumchlorid in DMF gelöst und sechs Stunden unter Rückfluß gekocht. Man entfernt das Lösungsmittel, löst den Rückstand in Wasser und extrahiert mit Essigester und anschließend mit Chloroform. Die Chloroformphasen wurden vereinigt, getrocknet, filtriert und am Rotationsverdampfer eingeengt.

Das Produkt wird dünnschichtchromatographisch über Amino-Kieselgel mit Acetonitril/H₂O 10/1 gereinigt (Rf = 0,58).

### Beispiel 3

### Synthese von Bis (bisethylenglycol-bipyridin) -4(4(4'-methyl-2,2'bipyridyl))-butansäure

3,0 g des in Beispiel 2 hergestellten Ruthenium-Komplexes wurden in 240 ml eines Ethanol-Wasser-Gemisches unter Argon gelöst. Es werden 0,82 g Bipyridyl-butansäure-Derivat hinzugefügt und drei Stunden unter Rückfluß erhitzt. Die Lösung wird eingeengt, mit Essigester gewaschen und mit Chloroform extrahiert. Man rotiert ein und reinigt den Rückstand über SP-Sephadex (Eluens: NaCl/HCl in Wasser).
Ausbeute: 500 mg, Reinheit (HPLC): 93 %
MS (PosLIMS) : 1455,5 = Ru²⁺Komplex PF₆⁻

### Beispiel 4

### Herstellung eines hydrophilen Metallkomplex-Aktivesterderivats

260 mg des in Beispiel 3 hergestellten Komplexes wurden in Methylenchlorid gelöst und mit equimolaren Mengen an Dicyclohexylcarbodiimid/N-Hydroxysuccinimidester versetzt. Man läßt zwölf Stunden rühren, filtriert DCH ab und rotiert ein. Das Rohprodukt wird über präparative HPLC gereinigt. Die Ausbeute ist 85 %.

### Beispiel 5

### Herstellung eines hydrophilen Metallkomplex-Maleimid-Derivats

150 mg des Metallkomplexes Ru(bipyridin)2(bipyridin-C0-N-hydroxysuccinimidester) gemäß EP-A-0 580 979 werden zusammen mit 100 mg Maleimido-Amino-Dioxaoctan (MADOO) und Triethylamin in Methylenchlorid ca. 12 h umgesetzt. Das Reaktionsgemisch wird 3 mal mit Wasser ausgeschüttelt und der Rückstand aus der organischen Phase über eine Sephadex-LH20-Säule mit Methylenchlorid/Methanol gereinigt. Man erhält die in Abb. 1a dargestellte Verbindung Ru(bpy)₂(bpy-C0-MADOO).
MS: M⁺ = 1025,3 (entspricht Ru²⁺ PF₆⁻-Komplex)

### Beispiel 6

### Herstellung eines hydrophilen Metallkomplex-Aktivester-Derivats

0,5 mmol des in Beispiel 5 als Ausgangsmaterial verwendeten Rutheniumkomplexes in 20 ml Dichlormethan werden mit 0,5 mmol Mono-Boc-Diaminodioxaoctan in 20 ml Dichlormethan und einem Äquivalent an Triethylamin umgesetzt. Die Aufreinigung erfolgt wie in Beispiel 5 beschrieben. Die Boc-Schutzgruppe wird nach Standardmethoden (Trifluoressigsäure/Methylenchlorid) abgespalten.

Das resultierende Produkt wird mit einer äquimolaren Menge an Korksäure-bis-N-hydroxysuccinimidester in Dimethylformamid für 2 h bei Raumtemperatur umgesetzt. Das Lösungsmittel wird entfernt, der Rückstand in Wasser aufgenommen und lyophilisiert. Das resultierende Produkt ist in Abb. 1b dargestellt.
- H-NMR:: 7,2 - 8,9 ppm : Bipyridin (22H);
2,8 ppm NHS-Ester (4H).

### Beispiel 7

### Synthese eines Metallkomplex-Hapten-Konjugates

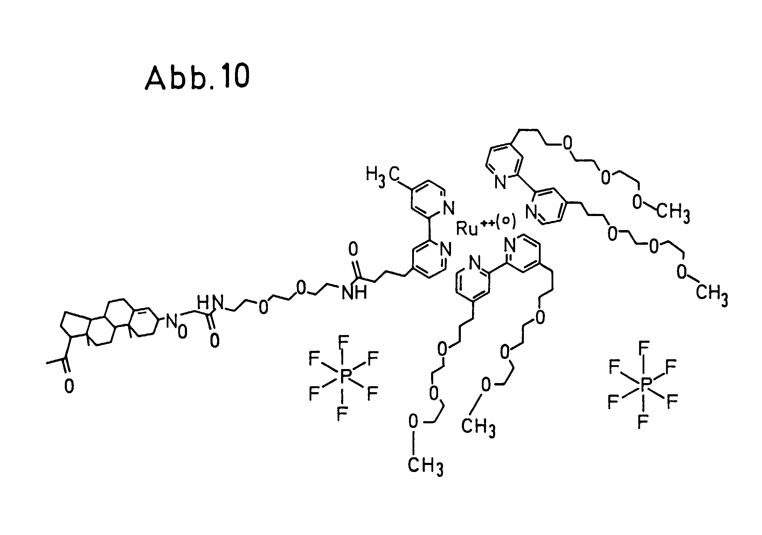

10 mg des N-Hydroxysuccinimidesters aus Beispiel 4 werden mit 3,2 mg Progesteron-3-carboxymethyl-oxim-diamondioxyoctan in 2 ml Methylenchlorid gelöst, 1,2 µl Triethylamin hinzugefügt und zwölf Stunden bei Raumtemperatur gerührt. Das Lösungsmittel wird entfernt und der Rückstand über Sephadex gereinigt. Das resultierende Konjugat ist in Abb. 10 dargestellt.
- MS (posLIMS):: M+ = 1923,0 (Ruthenium-Komplex-Progesteron-Konjugat²⁺ Trifluoracetat

### Beispiel 8

### Herstellung von Metallchelat-markierten Peptiden

Die Metallchelat-markierten Peptide wurden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu wurden jeweils 4.0 Äquivalente der in Tabelle 1 dargestellten Aminosäurederivate verwendet:

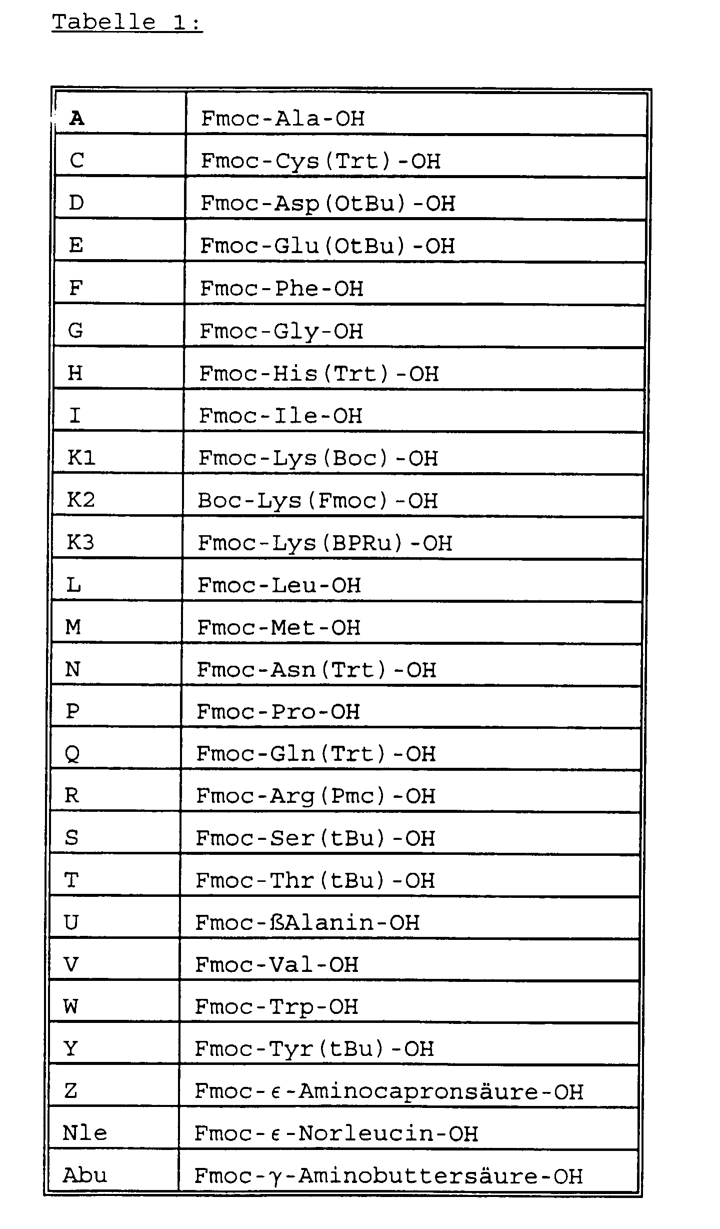

Bei der Variante (a) - Einführung der Markierung nach Beendigung der Festphasensynthese - wurde ein aktivierter hydrophiler Ruthenium(bipyridyl)₃-Komplex (BPRu) an die N-terminale Aminosäure des Peptids gekoppelt. Das Lysin-Derivat K1 wurde für den Spacerbereich und das Lysin-Derivat K2 für den Epitopbereich verwendet.

Gemäß Variante (b) erfolgte die Einführung von Metallchelatgruppen in die Peptidsequenz durch direkten Einbau von Metallchelat-gekoppelten Aminosäurederivaten, z.B. innerhalb der Sequenz über einen mit Metallchelat-Aktivester ε-derivatisierten Lysinrest, z.B. das Lysin-Derivat K3 oder N-terminal durch Verwendung eines α-derivatisierten Aminosäurerests.

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Bei Anwesenheit von Cysteinresten in der Peptidsequenz erfolgte unmittelbar nach Beendigung der Synthese eine Oxidation an der Festphase mit Jod in Hexafluorisopropanol/Dichlormethan.

Die Freisetzung des Peptids vom Träger und die Abspaltung der säurelabilen Schutzgruppen erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschließend mit 300 ml gekühltem Diisopropylether versetzt und zur vollständigen Fällung des Peptids 40 min bei 0°C gehalten. Der Niederschlag wurde abfiltriert, mit Diisopropylether nachgewaschen, mit wenig 50 %-iger Essigsäure gelöst und lyophilisiert. Das erhaltene Rohmaterial wurde mittels präparativer HPLC an Delta-PAK RP C18-Material (Säule 50 x 300 mm, 100 Å, 15 µ) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierten Materials wurde mittels Ionenspray-Massenspektrometrie geprüft.

Die Einführung der Metallchelat-Markierung erfolgte gemäß Variante (a) über entsprechende Aktivester-Derivate an die freie N-terminale Aminogruppe des trägergebundenen Peptids. Hierzu wurden 4 Äquivalente hydrophiler Ruthenium(bipyridyl)₃-Komplexe (BPRu) pro freie primäre Aminofunktion, aktiviert mit N-Hydroxybenzotriazol/Dicyclohexylcarbodiimid und in wenig DMSO gelöst, zugetropft und bei Raumtemperatur gerührt. Der Umsatz wurde über analytische HPLC verfolgt. Nach Abspaltung vom Träger wurde das Produkt mittels präparativer HPLC aufgereinigt. Die Identität des eluierten Materials wurde mittels Ionenspray-Massenspektrometrie geprüft.

Die Herstellung der Peptide erfolgte auch durch eine Kombination von Variante (a) und (b), d.h. Einbau von Metallchelat-gekoppelten Aminosäurederivaten innerhalb der Sequenz, Abspaltung der N-terminalen Fmoc-Gruppe und Reaktion der freien N-terminalen Aminogruppe mit einem Metallchelat-Aktivesterderivat.

Bei einem ausschließlich direkten Einbau der Metallchelatgekoppelten Aminosäurederivate während der Festphasensynthese gemäß Variante (b) war eine nachträgliche Einführung von Metallchelat-Aktivestern nicht mehr erforderlich.

Aus den Bereichen gp120, gp41 und gp32 von HIVI bzw. HIVII wurden die in Tabelle 2 dargestellten Peptid-Metallkomplex-Konjugate hergestellt.

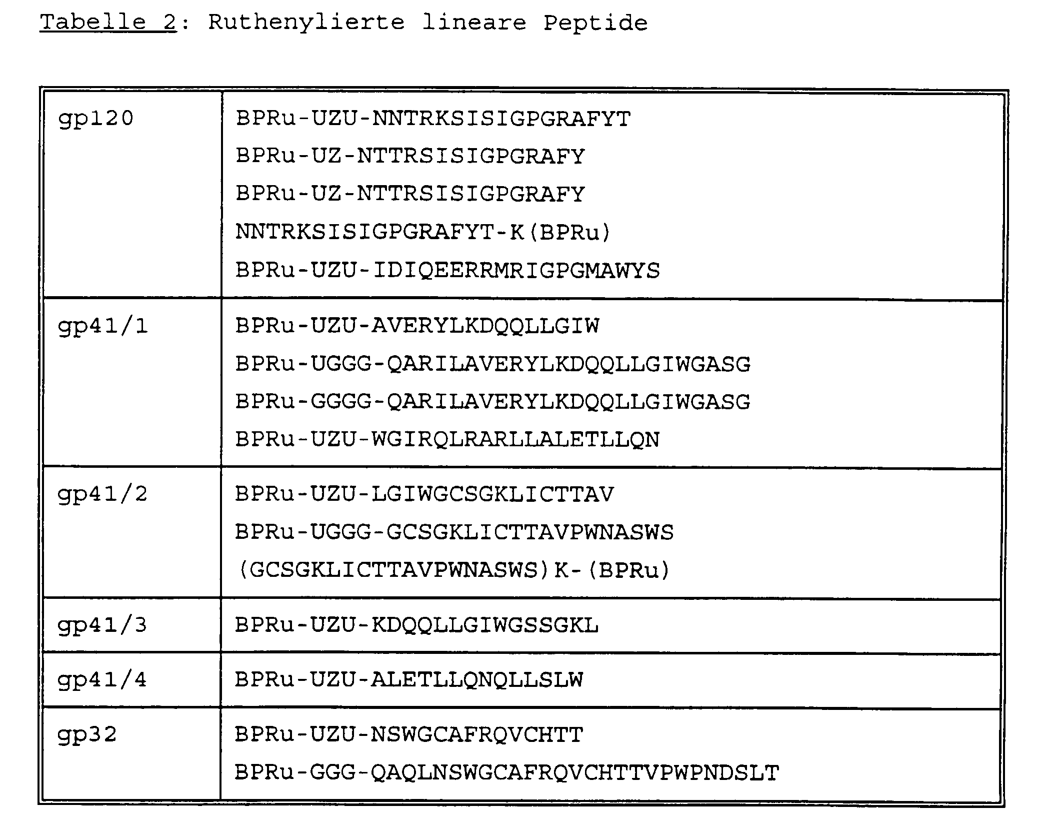

Aus dem NS5-Bereich, dem NS4-Bereich und dem Core-Bereich von HCV wurden die in der folgenden Tabelle 3 dargestellten Peptide synthetisiert.

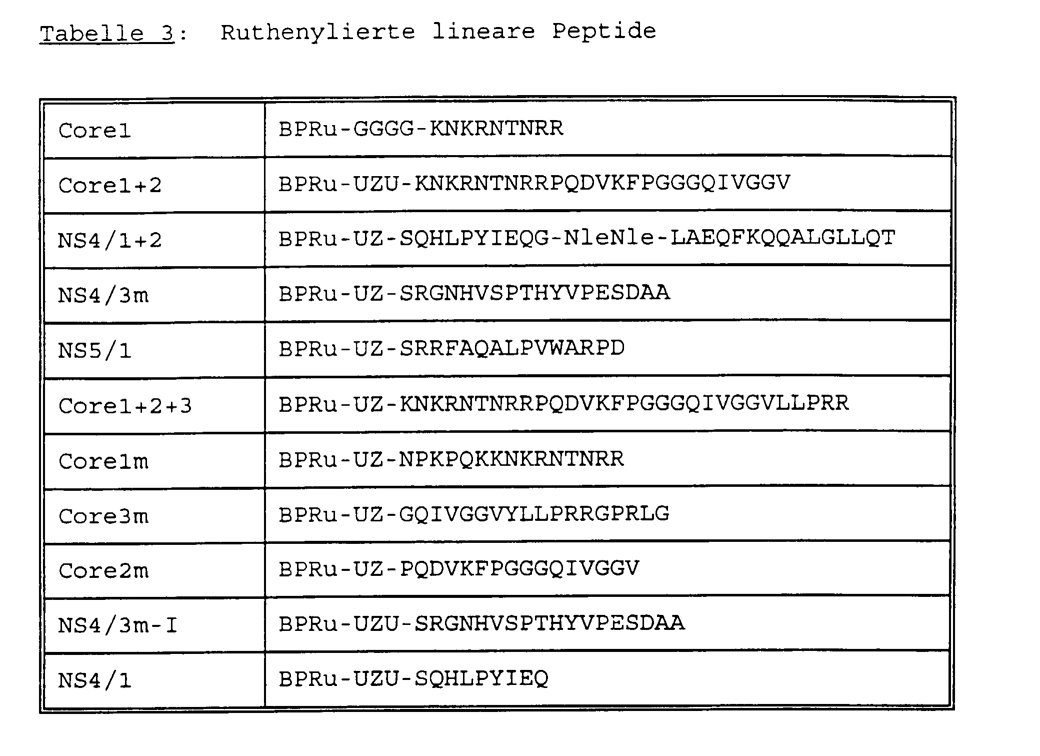

Die Herstellung Biotin-markierter Peptide erfolgte entweder N-terminal durch eine Derivatisierung am Harz (Biotin-Aktivester) oder in die Sequenz über einen mit Biotinaktivester-ε-derivatisierten Lysinrest (Fmoc-Lys (Biotin)-OH).

### Beispiel 9

### Herstellung von Diethyl-α,α,α',α'-tetrakis(ethoxycarbonyl)-2,2'-bipyridin-4,4'-diyl-dipropionat (entsprechend Verbindung (1) in Abb. 7)

Zu einer Mischung aus 6,85 g( 29,5 mmol) Triethyl-methantricarboxylat und 4,1 g (29,7 mmol) Kaliumcarbonat in 50 ml Toluol/DMF (3/2) tropft man 2,00 g (5,8 mmol) 4,4'-Bis(brommethyl)-2,2,'-bipyridin in 40 ml Toluol/DMF (3/2) bei 50°C unter gutem Rühren zu. Man rührt weitere 4 Tage bei 65°C filtriert und entfernt anschließend das Lösungsmittel im Vakuum. Der ölige Rückstand wird in 100 ml Toluol aufgenommen und nacheinander 3 mal mit Wasser, 3 mal mit 7 % Natronlauge und 3 mal mit Wasser ausgeschüttelt. Die organische Phase wird gesammelt und über Natriumsulfat getrocknet. Nach Entfernen der flüchtigen Bestandteile im Vakuum wird der Rückstand aus Cyclohexan umkristallisiert. Zur vollständigen Entfernung von Verunreinigungen wird säulenchromatographisch getrennt (SiO₂, CHCI₃/MeOH (10:1) erste Bande).
Farblose Kristalle (Cyclohexan)
Ausbeute: 2,95 g (79 %)
Schmp.: 116 °C

¹H-NMR (250 MHz, CDCI₃, 25 °C): δ=1,21 (t, 18H, ³J=7,2 Hz), 3,57 (s, 4H), 4,22 (q, 12H, ³J=7,2 Hz), 7,25 (dd, 2H, ³J=5,1 Hz, ⁴J=1,2 Hz), 8,29 (d, 2H, ⁴J=1,2 Hz), 8,51 (dd,2H, ³J=5,1 Hz, ⁴J=1,2 Hz)
¹³C-NMR (75 MHz, CDCI₃, 25 °C): δ=13,96 (CH₃), 38,1 (CH₂), 62,6 (H₂CO), 66,5 (CCO), 123,4, 123,6, 145,9, 149,0, 155 (Pyridin-C, CH), 166,5 (C=O).
IR (KBr/fest) [cm⁻¹]: 556, 610, 863, 1026, 1186, 1258, 1305, 1594, 1737 vs. 2988
MS-50: (180 °C, 70 eV, 300 µA, m/e): gef.: 644,2585
C₃₂H₄₀N₂O₁₂ (644,682)

### Beispiel 10

### Herstellung von N,N'-Bis(2-hydroxy-1,1-bis(hydroxymethyl)ethyl)-α,α-bishydroxy-2,2'-bipyridin-4,4'-diyldipropionamid (entsprechend Verbindung (2) in Abb. 7)

Zu einer Lösung aus 752,7 mg(1,17 mmol) des Hexaesters (1) aus Beispiel 7 und 848,6 mg (7,00 mmol) α,α,α-Tris-(hydroxymethyl)-methylamin in 10 ml über CaH₂ getrocknetem DMSO gibt man unter Rühren 967,5 mg (7,00 mmol) Kaliumcarbonat. Nach Zugabe der Base wird die Mischung leicht gelblich. Nach weiterem 10 stündigen Rühren bei 25°C wird die Suspension zentrifugiert und die Lösung vom festen Kaliumcarbonat abdekantiert. Das Lösungsmittel wird im Vakuum (0,001 mbar) bei 30 °C entfernt. Der gelbliche, ölige Rückstand wird in wenig Wasser suspendiert und das Produkt durch langsame Zugabe von trockenem Aceton (über P₄O₁₀ destilliert) ausgefällt. In der Kälte erfolgt die vollständige Ausfällung. Man dekantiert die Lösung ab und trocknet den Rückstand mehrere Tage über P₄O₁₀. Zurück bleibt ein hygroskopischer farbloser Feststoff, der ohne weitere Reinigung eingesetzt wird.
Ausbeute: 0,652 g (67 %)
¹H-NMR (250 MHz, DMSO-d₆, 25 °C): δ=3,08 (d, 4H), 3,22 (s, 8H), 3,47 (d, 8H, ²J=10,8 Hz), 3,55 (d, 8H, ²J=11,1Hz), 3,69 (t, 2H), 4,6-5,1 (bs, OH), 7,27 (d, 2H, Pyridyl-H, ³J=4,8 Hz), 7,4-7,7 (s, NH), 8,23 (s, 2H, Pyridyl-H), 8,51 (d, 2H, Pyridyl-H, ³J=4,8 Hz)
¹³C-NMR (75 MHz, DMSO-d₆, 25 °C): δ=30,7 (CH₂), 59,7 (CH₂OH), 61,7 (CR₄), 63,34 (CR₄), 78,5 (CCO), 122,8, 126,0, 146,0, 148,7, 155,0 (Pyridin-C,CH₂), 170,2 (CONH)
IR: (Kbr/fest) [cm⁻¹]: 3336, 2936, 2880, 1675, 1597, 1559, 1533, 1465, 1363, 1051 (vs)
FAB^{•}-MS (m-NBA, m/e): 833,3, 855,3, 871,3, 965,2 (M+H)⊕, (M+Na)^{⊕} , (M+K)^{⊕}, (M+Cs)^{⊕}
C₃₄H₅₂N₆O₁₈ (832,3)

### Beispiel 11

### Herstellung von halbkäfig- bzw. käfigförmigen hydrophilen Liganden gemäß Reaktionsschema III (Abb. 9a und b)

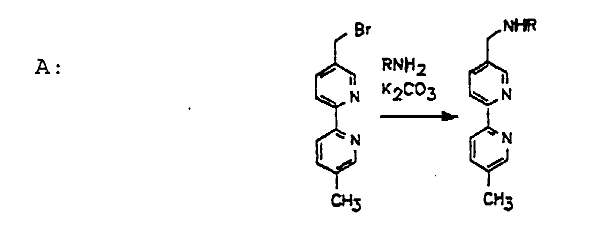

- Ansatz:: 2,6 g (10 mmol) Bipyridin-methylbromid
80 ml 2-Methoxyethylamin
10 g Kaliumcarbonat

### Durchführung:

Das Bromid wurde unter Rühren zu einer Suspension von gepulvertem Kaliumcarbonat in 2-Methoxyethylamin gegeben. Die Suspension wurde dann 12 h bei Raumtemperatur gerührt. Anschließend filtrierte man ab, destillierte das überschüssige 2-Methoxyethylamin ab und trocknete den Rückstand im Vakuum. Der Rückstand wurde chromatographiert (SiO₂; CH₂Cl₂/CH₃OH/NH₃, 100:10:1). Man erhielt ein hellgelbes Öl.
Ausbeute: 1,06 g (3,85 mmol) 38 %
¹H-NMR (250 MHz, CDCI₃): 1,95 (s, 1H, NH); 2,26 (s, 3H, Pyridyl-CH₃), 2,7 (t, ³J = 5,28 Hz, 2H, OCH₂); 3,24 (s, 3H, OCH₃); 3,41 (t, ³J = 5,28 Hz, 2H, NCH₂); 3,76 (s, 2H, Pyridyl-CH₂), 7,5 (dd, ³J = 8,35 Hz, ⁴J = 2,17 Hz, 1H, Pyridyl H); 7,69 (dd, ³J = 8,19 Hz, ⁴J = 2,24 Hz, 1 H), Pyridyl H); 8,17 (d, ³J = 8,02 Hz, 1H, Pyridyl-H); 8,22 (d, ³J = 8,24 Hz, 1 H, Pyridyl-H); 8,39 (d⁴J = 1,84 Hz, 1H, Pyridyl H) ; 8,85 (d, ⁴J = 1,85 Hz, 1 H, Pyridyl H) ppm.

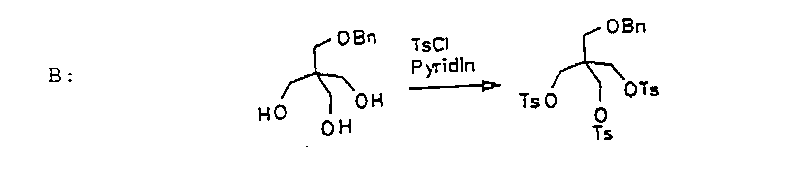

- Ansatz:: 2,26 (10 mmol) Tri-alkohol
6,67 (35 mmol) Tosylchlorid

### Durchführung:

Eine Lösung des Tri-alkohols in 20 ml Pyridin wurde langsam mit einer Lösung von Tosylchlorid in 20 ml Pyridin unter Kühlen, Schutzgas und Rühren versetzt, so daß die Temperatur der Reaktionsmischung 10 °C nicht überschritt. Dann wurd enoch 24 h bei Raumtemperatur gerührt. Anschließend goß man vorsichtig auf eine Mischung von 10 ml Wasser, 20 ml Methanol und 8 ml konz. Salzsäure. Ds ausgefallene Produkt bzw. abgeschiedene Öl wurde abfiltriert bzw. abgetrennt und chromatographisch gereinigt (SiO₂; CH₂Cl₂/CH₃OH/NH₃, 100:10:1). Man erhielt farblose Kristalle.
Smp.: 57 - 59 °C
¹H-NMR (250 MHz, CD₂Cl₂): 2,4 (s, 9H, Ar-CH₃); 3,25 (s, 2H, CH₂); 3,88 (s, 6H, CH₂); 4,22 (s, 2H, CH₂); 7,02-7,1 (m, 2H, Ar-H); 7,25-7,3 (m, 3H, Ar-H); 7,31 (d, ³J = 6,46 Hz, 6H, Ar-H); 7,31 (d, ³J = 6,46 Hz, 6H, Ar-H); 7,67 (d, ³J = 6,46 Hz, Ar-H)ppm
¹³C-NMR u. DEPT-135 (62,8 MHz, CDCI₃); 145,36; 137,20; 131,75; 43,82 (C_{q}); 130,05; 128,39; 128,33; 127,92; 127,75; 127,23 (CH); 73,28, 66,71; 66,31 (CH₂); 21,67 (CH₃) ppm

### Beispiel 13

### Anwendung hydrophiler Metallkomplexe in immunologischen Tests

Es wurde ein Doppel-Antigen-Brückentest zum Nachweis spezifischer Antikörper gegen Hepatitis C-Virus (HCV) durchgeführt. Hierbei wurde die Probeflüssigkeit mit einem Ruthenium-markierten Antigen und einem biotinylierten Antigen gegen den zu bestimmenden Antikörper in Gegenwart einer Streptavidin-beschichteten Festphase inkubiert. Das Vorhandensein von Anti-HCV-Antikörpern in der Probeflüssigkeit wurde durch Bestimmung der Markierung in der Festphase durch Elektrochemilumineszenz nach dem Flash-System bestimmt.

Als Antigen wurde ein HCV-Polypeptid, welches die Aminosäuren 1207-1488 von HCV enthält, verwendet. Die Aminosäuresequenz und Herstellung eines derartigen Polypeptids ist in DE-A-44 28 705.4 beschrieben.

Zur Derivatisierung des HCV-Polypeptids mit Succinimidesteraktivierten Rutheniumkomplexen wurde das lyophilisierte Polypeptid in einem 100 mM Natriumphosphatpuffer pH 6,5, 0,1 % SDS in einer Proteinkonzentration von 10 mg/ml gelöst. Durch Zusatz von 5 M wurde der pH-Wert auf 8,5 eingestellt und die Lösung mit Dithiothreitol auf eine Endkonzentration von 2 mM abgestoppt. Zu dieser Lösung wurde die der gewünschten Angebotsstöchiometrie entsprechende Menge eines Succinimidester-aktivierten Rutheniumkomplexes in DMSO zugegeben und anschließend für 60 min bei 65 °C unter Rühren inkubiert. Die Reaktion wurde durch Aufstocken des Reaktionsgemisches mit Lysin auf eine Endkonzentration von 10 mM und eine weitere Inkubation für 30 min abgestoppt. Anschließend wurde der Ansatz gegen 100 mM Natriumphosphatpuufer pH 6,5, 0,1 % SDS dialysiert. Die resultierende Proteinlösung wurde mit Saccharose (Endkonzentration 6,5 % (w/v)) versetzt und in Portionen lyophilisiert.

Zur Herstellung eines mit einem Maleinimid-aktivierten Rutheniumkomplex derivatisierten HCV-Polypeptids wurde das lyophilisierte Protein in 100 mM Natriumphosphatpuffer pH 6,5, 0,1 % SDS (Proteinkonzentration 10 mg/ml) aufgenommen. Zu dieser Lösung wurde eine der gewünschten Angebotsstöchiometrie entsprechende Menge des Maleinimid-aktivierten Rutheniumkomplexes in DMSO zugegeben und 60 min bei 25°C unter Rühren inkubiert. Die Reaktion wurde durch Aufstocken des Reaktionsgemisches mit Cystein auf eine Endkonzentration von 10 mM und weitere Inkubation für 30 min abgestoppt. Das Reaktionsgemisch wurde daraufhin wie oben beschrieben dialysiert, mit Saccharose versetzt und in Portionen lyophilisiert.

Es wurden 3 Experimente durchgeführt, in denen jeweils unterschiedliche ruthenylierte Antigene eingesetzt wurden. Für Experiment A (Vergleich) wurde der in den Beispielen 5 und 6 als Ausgangsmaterial verwendete Ruthenium-Komplex gemäß EP-A-0 580 979 in einem stöchiometrischen Verhältnis von 1:3 an das Polypeptid gekoppelt. Für Experiment B wurde das Polypeptid mit dem in Beispiel 5 hergestellten erfindungsgemäßen hydrophilen Ruthenium-Komplex in einem stöichiometrischen Verhältnis von 1:3 gekoppelt. Für Experiment C wurde das Polypeptid mit dem in Beispiel 6 hergestellten hydrophilen Ruthenium-Komplex im stöichiometrischen Verhältnis von 1:1 gekoppelt. Als biotinyliertes Antigen wurde in allen 3 Experimenten ein Polypeptid verwendet, das im stöichiometrischen Verhältnis von 1:6 an ein Maleimid-aktiviertes Biotin gekoppelt worden war. Das ruthenylierte und das biotinylierte Antigen wurden jeweils in einer Konzentration von 400 ng/ml Testflüssigkeit eingesetzt.

In Tabelle 4 ist das Ergebnis der Experimente A, B und C in ECL-Counts dargestellt. Es ist ersichtlich, daß erst die Verwendung der erfindungsgemäßen hydrophilen Metallkomplexe als Markierungsgruppen eine zuverlässige Unterscheidung zwischen einer negativen Serumprobe und einer kritischen positiven Serumprobe erlaubt. Dies zeigt sich in einem höheren Verhältnis positiv/negativ.

**Tabelle 4**

| Experiment | A (Vergleich) | B | C |
|---|---|---|---|
| negative Probe | 323317 | 84584 | 44274 |
| positive Probe | 465769 | 346734 | 313185 |
| Verhältnis positiv/negativ | 1,4 | 4 | 7 |

## Patentansprüche

1. Metallkomplexe mit der allgemeinen Formel (I):
[M(L₁L₂L₃)]ₙ - Xₘ A (I)
worin
- M ein zwei- oder dreiwertiges Metallkation ausgewählt aus Seltenerde- oder Übergangsmetallionen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- X eine reaktive oder aktivierbare funktionelle Gruppe ist, die an mindestens einen der Liganden L₁, L₂, L₃ kovalent gebunden ist,
- n eine ganze Zahl von 1 bis 10 ist,
- m eine ganze Zahl von 1 bis 6 ist und
- A eine oder mehrere zum Ladungsausgleich erforderliche, negativ geladene Gruppen bedeutet,
**dadurch gekennzeichnet,**
daß der Komplex mindestens eine hydrophile Gruppe ausgewählt aus C₂-C₃-Alkylenoxy-Einheiten,C₂-C₃-Alkylenthio-Einheiten,C₂-C₃-Alkylenamino-Einheiten oder/und Polyhydroxy-Einheiten enthält, mit der Maßgabe, daß die Gruppe X keine Hydroxy-, Methoxy-, 4-Methoxyphenoxy-, Dioxolan- oder CO₂CH₃-Gruppe ist, wobei die hydrophilen Gruppen Bestandteile von Substituenten der Liganden des Metallkomplexes sind und in einem Linker zwischen einem der Liganden und der Gruppe X oder/und in einfachen, nicht die Gruppe X enthaltenden Substituenten vorliegen.

2. Komplex nach Anspruch 1, dadurch gekennzeichnet, daß das Metallkation M ein Ruthenium-, Rhenium-, Osmium-, Chrom- oder Iridiumion ist.

3. Komplex nach Anspruch 1 oder 2, dadurch gekennzeichnet, daß das Metallkation M ein Rutheniumion ist.

4. Komplex nach einem der Ansprüche 1 bis 3, dadurch gekennzeichnet, daß die Liganden L₁, L₂, L₃ Bipyridin- oder/und Phenanthrolin-Ringsysteme enthalten.

5. Komplex nach einem der Ansprüche 1 bis 4, dadurch gekennzeichnet, daß die reaktive oder aktivierbare funktionelle Gruppe X ein Carbonsäurehalogenid, ein Carbonsäureanhydrid, ein Aktivester, ein Maleimid, ein Amin, eine Carbonsäure, ein Thiol, ein Halogenid oder eine photoaktivierbare Gruppe ist.

6. Komplex nach einem der Ansprüche 1 bis 5, dadurch gekennzeichnet, daß die negativ geladenen Gruppen A Hexafluorophosphat-, Trifluoracetat-, Tetrafluoroborat-Gruppen oder Halogenidionen sind.

7. Komplex nach einem der Ansprüche 1 bis 6, dadurch gekennzeichnet, daß die Polyhydroxy-Einheiten aus Gruppen der Formeln (IIa) oder (IIb) ausgewählt sind:
-NR-W (IIa) -O-W (IIb)
worin W einen organischen Rest mit mindestens 2 Hydroxygruppen und R Wasserstoff oder C₁-C₅-Alkyl bedeutet.

8. Komplex nach Anspruch 7, dadurch gekennzeichnet, daß die Polyhydroxy-Einheiten die Formel -NR-C(CH₂OH)₃ aufweisen.

9. Komplex nach einem der Ansprüche 1 bis 8, dadurch gekennzeichnet, daß die C₂-C₃-Alkylenoxy-, C₂-C₃-Alkylenthio bzw. C₂-C₃-Alkylenamino-Einheiten Ethylenoxy-, Ethylenthio-, bzw. Ethylenamino-Einheiten sind.

10. Komplex nach einem der Ansprüche 1 bis 9,
dadurch gekennzeichnet,
daß 1 bis 30 C₂-C₃-Alkylenoxy-,C₂-C₃-Alkylenthio- bzw. C₂-C₃-Alkylenamino-Einheiten pro Metallkation vorhanden sind.

11. Komplex nach einem der Ansprüche 1 bis 10 mit der allgemeinen Formel (III): 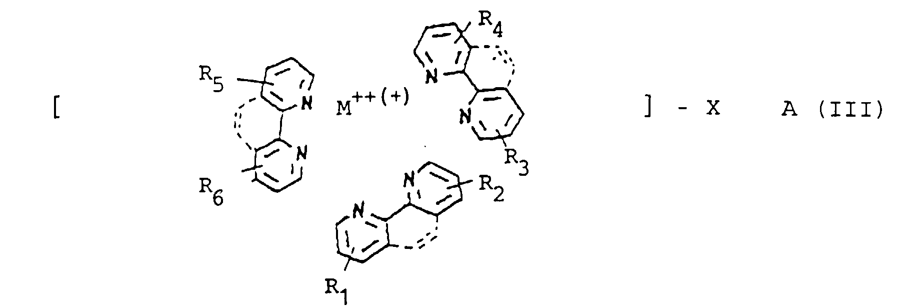 worin M, X und A wie in Anspruch 1 definiert sind, R₁, R₂, R₃, R₄, R₅ und R₆ gleich oder verschieden sind und jeweils einen oder mehrere Substituenten bedeuten, unter der Voraussetzung, daß X über einen der Substituenten R₁, R₂, R₃, R₄, R₅ oder R₆ an einen der Liganden gebunden ist und daß mindestens einer der Substituenten R₁, R₂, R₃, R₄, R₅ oder R₆ mindestens eine hydrophile Gruppe, ausgewählt aus C₂-C₃-Alkylenoxy-, C₂-C₃-Alkylenthio- und C₂-C₃-Alkylenamino-Einheiten enthält.

12. Komplex nach Anspruch 11 mit der allgemeinen Formel (IIIa) : 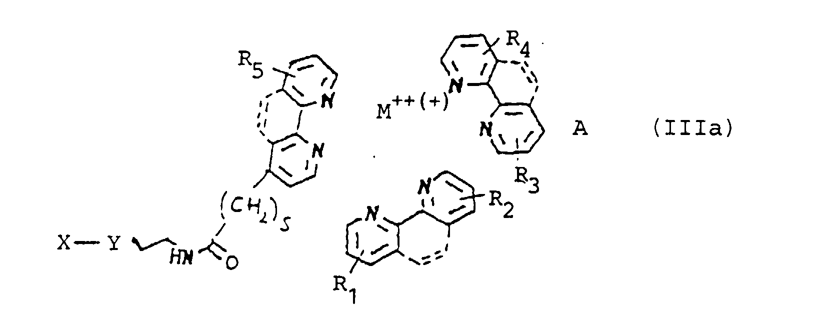 worin M, X und A wie in Anspruch 1 definiert sind, R₁, R₂, R₃, R₄ und R₅ wie in Anspruch 11 definiert sind, s eine ganze Zahl von 0 bis 6 ist und Y eine hydrophile Gruppe mit 1 bis 10 hydrophilen Einheiten bedeutet, ausgewählt aus C₂-C₃-Alkylenoxy-, C₂-C₃-Alkylenthio und C₂-C₃-Alkylenamino-Einheiten.

13. Komplex nach Anspruch 1 mit der allgemeinen Formel (IIIb) : 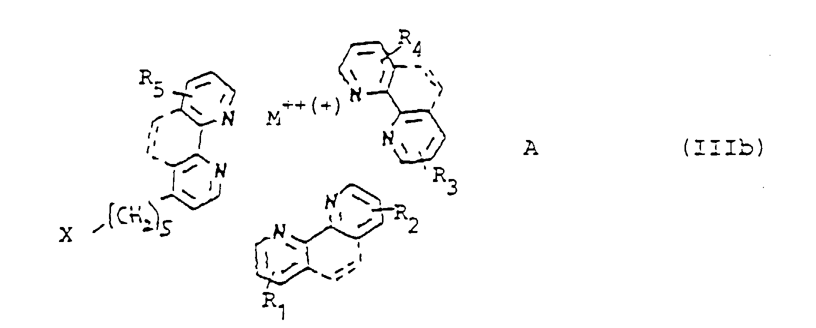 worin M, X und A wie in Anspruch 1 definiert sind, R₁, R₂, R₃, R₄ und R₅ wie in Anspruch 11 definiert sind und s eine ganze Zahl von 0 bis 6 ist, unter der Voraussetzung, daß R₁, R₂, R₃, R₄ oder/und R₅ eine hydrophile Substituentengruppe enthalten, die jeweils 1 bis 10 C₂-C₃-Alkylenoxy-, C₂-C₃-Alkylenthio oder/und C₂-C₃-Alkylenamino-Einheiten umfaßt.

14. Komplex nach einem der Ansprüche 1 bis 10 mit der allgemeinen Formel (IV) : 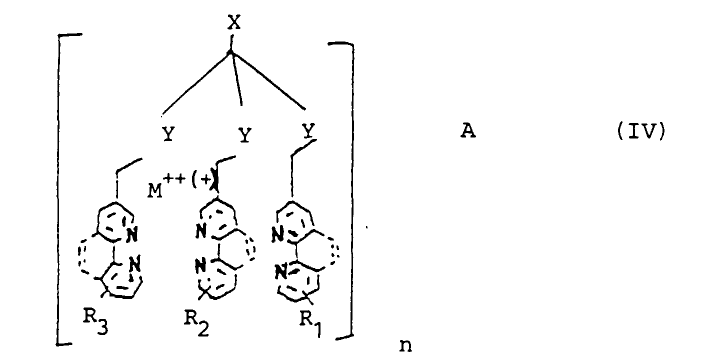 worin M, X, n und A wie in Anspruch 1 definiert sind, R₁, R₂ und R₃ gleich oder verschieden sind und jeweils einen oder mehrere Substituenten bedeuten und Y jeweils eine hydrophile Linkergruppe bedeutet, die 1 bis 10 hydrophile Einheiten, ausgewählt aus C₂-C₃-Alkylenoxy-, C₂-C₃-Alkylenthio- und C₂-C₃-Alkylenamino-Einheiten umfaßt.

15. Komplex nach einem der Ansprüche 1 bis 10 mit der allgemeinen Formel (V) : 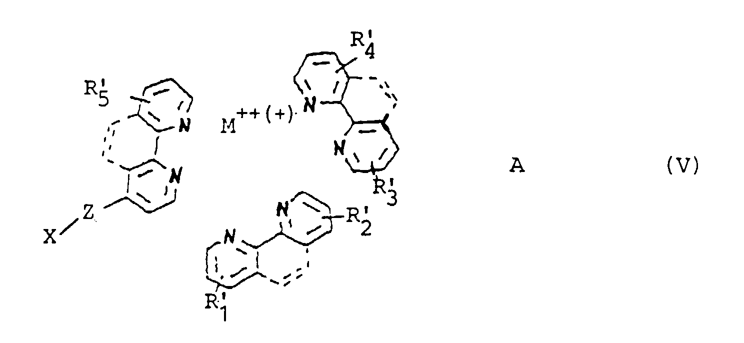 worin M, X und A wie in Anspruch 1 definiert sind, Z eine Linkergruppe bedeutet, R'₁, R'₂, R'₃, R'₄ oder/und R'₅ gleich oder verschieden sind und jeweils einen oder mehrere Substituenten bedeuten, unter der Voraussetzung, daß R'₁, R'₂, R'₃ oder/und R'₄ mindestens eine hydrophile Substituentengruppe enthält, die eine Polyhydroxy-Einheit umfaßt.

16. Komplex nach Anspruch 15, dadurch gekennzeichnet, daß die OH-Gruppen der Polyhydroxy-Einheiten durch weitere hydrophile Gruppen substituiert sind.

17. Komplex nach Anspruch 16, dadurch gekennzeichnet, daß die weiteren hydrophilen Gruppen ein Dendrimer bzw. ein dendrimeres Bauelement der Formel (VIa) oder (VIb) umfassen:
-A₁-NR-W₁ (A₂-NR-W₂)_{n'} (VIa)
-A₁-O-W₁(A₂-O-W₂)_{n'} (VIb)
worin
- A₁ und A₂ gleich oder verschieden sind und Linkergruppen bedeuten,
- W₁ und W₂ gleich oder verschieden sind und einen organischen Rest mit mindestens 2 Hydroxygruppen bedeuten,
- R Wasserstoff oder einen C₁-C₅-Alkylrest bedeutet und
- n' 0 ist oder der Zahl der Hydroxygruppen von W₁ entspricht.

18. Konjugat umfassend eine biologische Substanz, an die mindestens ein Metallkomplex mit der allgemeinen Formel (l) kovalent gekoppelt ist:
[M(L₁L₂L₃)]ₙ - Xₘ A (I)
worin
- M ein zwei- oder dreiwertiges Metallkation ausgewählt aus Seltenerde- oder Übergangsmetallionen ist,
- L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
- X eine reaktive oder aktivierbare funktionelle Gruppe ist, die an mindestens einen der Liganden L₁, L₂, L₃ kovalent gebunden ist,
- n eine ganze Zahl von 1 bis 10 ist,
- m eine ganze Zahl von 1 bis 5 ist und
- A eine oder mehrere zum Ladungsausgleich erforderliche, negativ geladene Gruppe bedeutet,
**dadurch gekennzeichnet**,
daß der Komplex mindestens eine hydrophile Gruppe ausgewählt aus C₂-C₃-Alkylenoxy-Einheiten,C₂-C₃-Alkylenthio-Einheiten,C₂-C₃-Alkylenamino-Einheiten oder/und Polyhydroxy-Einheiten enthält, wobei die hydrophilen Gruppen Bestandteile von Substituenten der Liganden des Metallkomplexes sind und in einem Linker zwischen einem der Liganden und der Gruppe X oder/und in einfachen, nicht die Gruppe X enthaltenden Substituenten vorliegen.

19. Konjugat nach Anspruch 18, dadurch gekennzeichnet, daß die biologische Substanz Biotin, ein Antikörper oder Antikörperfragment, eine Nukleinsäure, ein Polypeptidantigen, ein immunologisch reaktives Peptid oder ein Hapten ist.

20. Verwendung von Metallkomplexen nach einem der Ansprüche 1 bis 17 oder Konjugaten nach Anspruch 18 oder 19 in einem immunologischen Nachweisverfahren.

21. Verwendung nach Anspruch 20 in einem Elektrochemilumineszenzverfahren.

22. Verwendung nach Anspruch 21,
**dadurch gekennzeichnet,**
daß man die Elektrochemilumineszenz-Messung in Gegenwart eines Reduktionsmittels für den Metallkomplex durchführt.

23. Verwendung nach Anspruch 22,
**dadurch gekennzeichnet**,
daß das Reduktionsmittel in die Ligandensphäre des Komplexes integriert ist.

## Claims

1. Metal complexes of the general formula (I):
[M(L₁L₂L₃)]ₙ - Xₘ A (I)
in which
- M is a divalent or trivalent metal cation selected from rare earth or transition metal ions,
- L₁, L₂ and L₃ are the same or different and denote ligands with at least two nitrogen-containing heterocycles wherein L₁, L₂ and L₃ are bound to the metal cation via nitrogen atoms,
- X is a reactive or activatable functional group which is covalently bound to at least one of the ligands L₁, L₂ and L₃,
- n is an integer from 1 to 10,
- m is an integer from 1 to 6 and
- A denotes one or several negatively charged groups that are required to balance the charge
wherein
the complex contains at least one hydrophilic group selected from C₂-C₃ alkyleneoxy units, C₂-C₃ alkylenethio units, C₂-C₃ alkyleneamino units or/and polyhydroxy units where the hydrophilic groups are components of substituents of the ligands of the metal complex and are present in a linker between one of the ligands and the group X or/and in single substituents which do not contain the group X.

2. Complex as claimed in claim 1, **wherein** the metal cation M is a ruthenium ion, rhenium ion, osmium ion, chromium ion or iridium ion.

3. Complex as claimed in claim 1 or 2,
**wherein** the metal cation M is a ruthenium ion.

4. Complex as claimed in one of the claims 1 to 3,
**wherein** the ligands L₁, L₂ and L₃ contain bipyridine or/and phenanthroline ring systems.

5. Complex as claimed in one of the claims 1 to 4,
**wherein** the reactive or activatable functional group X is a carboxylic acid halogenide, a carboxylic acid anhydride, an active ester, a maleimide, an amine, a carboxylic acid, a thiol, a halogenide or a group that can be photo-activated.

6. Complex as claimed in one of the claims 1 to 5,
**wherein** the negatively charged groups A are hexafluorophosphate, trifluoroacetate, tetrafluoroborate groups or halogenide ions.

7. Complex as claimed in one of the claims 1 to 6,
**wherein** the polyhydroxy units are selected from groups of formulae (IIa) or (IIb):
-NR-W (IIa) -O-W (IIb)
in which W denotes an organic residue with at least 2 hydroxy groups and R denotes hydrogen or C₁-C₅ alkyl.

8. Complex as claimed in claim 7,
**wherein** the polyhydroxy units have the formula -NR-C(CH₂OH)₃.

9. Complex as claimed in one of the caims 1 to 8,
**wherein** the C₂-C₃ alkyleneoxy units, C₂-C₃ alkylenethio units or C₂-C₃ alkyleneamino units are ethyleneoxy, ethylenethio or ethyleneamino units respectively.

10. Complex as claimed in one of the caims 1 to 9,
**wherein** 1 to 30 C₂-C₃ alkyleneoxy units, C₂-C₃ alkylenethio units or C₂-C₃ alkyleneamino units are present per metal cation.

11. Complex as claimed in one of the claims 1 to 10 of the general formula (III): 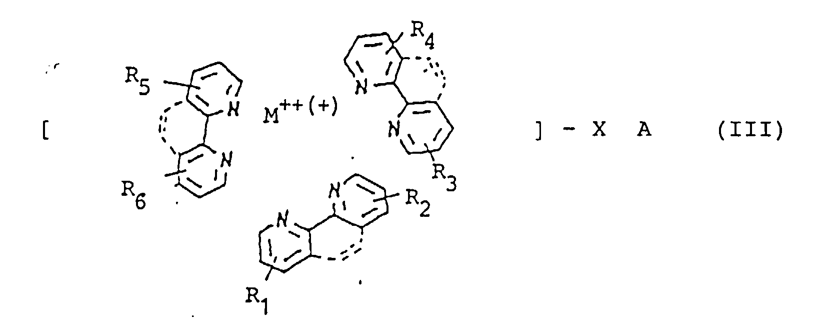 in which M, X and A are defined as in claim 1, R₁, R₂, R₃, R₄, R₅ and R₆ are the same or different and each denotes one or several substituents provided that X is bound to one of the ligands via one of the substituents R₁, R₂, R₃, R₄, R₅ or R₆ and that at least one of the substituents R₁, R₂, R₃, R₄, R₅ or R₆ contains at least one hydrophilic group selected from C₂-C₃ alkyleneoxy units, C₂-C₃ alkylenethio units and C₂-C₃ alkyleneamino units.

12. Complex as claimed in claim 11 having the general formula (IIIa) 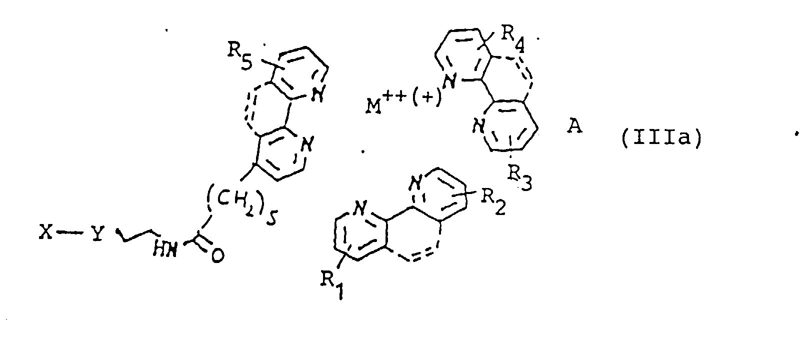 in which M, X and A are defined as in claim 1, R₁, R₂, R₃, R₄ and R₅ are defined as in claim 11, s is an integer from 0 to 6 and Y denotes a hydrophilic group with 1 to 10 hydrophilic units selected from C₂-C₃ alkyleneoxy units, C₂-C₃ alkylenethio units and C₂-C₃ alkyleneamino units.

13. Complex as claimed in claim 1 having the general formula (IIIb) 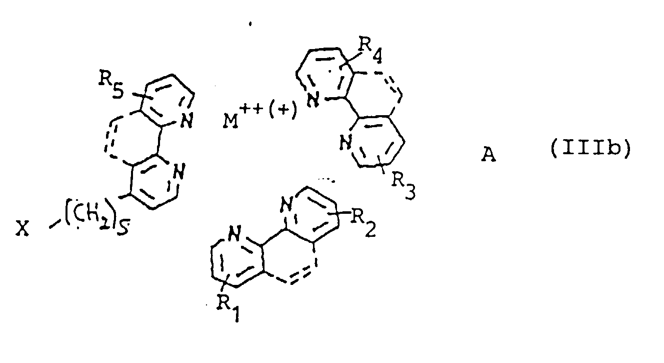 in which M, X and A are defined as in claim 1, R₁, R₂, R₃, R₄ and R₅ are defined as in claim 11, s is an integer from 0 to 6, provided that R₁, R₂, R₃, R₄ or/and R₅ contain a hydrophilic substituent group which each comprise 1 to 10 C₂-C₃ alkyleneoxy units, C₂-C₃ alkylenethio units or/and C₂-C₃ alkyleneamino units.

14. Complex as claimed in one of the claims 1 to 10 having the general formula (IV): 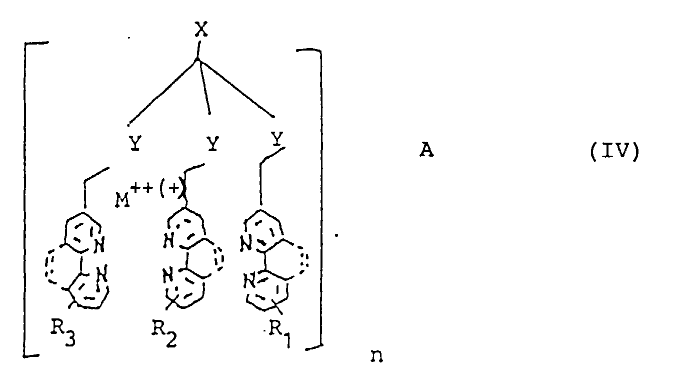 in which M, X, n and A are defined as in claim 1, R₁, R₂ and R₃ are the same or different and each denotes one or several substituents and Y in each case denotes a hydrophilic linker group which comprises 1 to 10 hydrophilic units selected from C₂-C₃ alkyleneoxy units, C₂-C₃ alkylenethio units and C₂-C₃ alkyleneamino units.

15. Complex as claimed in one of the claims 1 to 10 having the general formula (V) 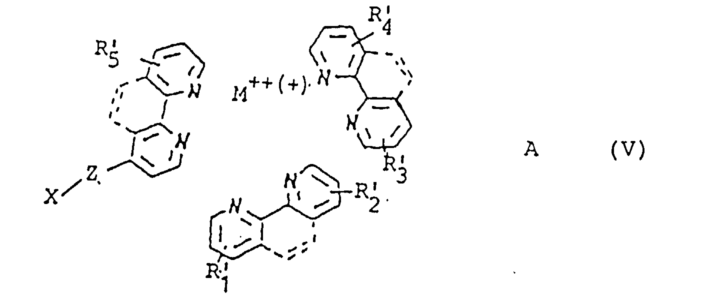 in which M, X and A are defined as in claim 1, Z denotes a linker group, R'₁, R'₂, R'₃, R'₄ or/and R'₅ are the same or different and each denotes one or several substituents, provided that R'₁, R'₂, R'₃ or/and R'₄ contain at least one hydrophilic substituent group which comprises a polyhydroxy unit.

16. Complex as claimed in claim 15,
**wherein** the OH groups of the polyhydroxy units are substituted by further hydrophilic groups.

17. Complex as claimed in claim 16,
**wherein** the further hydrophilic groups comprise a dendrimer or a dendrimeric structural element of formula (VIa) or (VIb):
-A₁-NR-W₁(A₂-NR-W₂)_{n'} (VIa)
-A₁-O W₁(A₂-O-W₂)_{n'} (VIb)
in which
- A₁ and A₂ are the same or different and denote linker groups
- W₁ and W₂ are the same or different and denote an organic residue with at least 2 hydroxy groups
- R denotes hydrogen or a C₁-C₅ alkyl residue and
- n' is 0 or corresponds to the number of hydroxy groups on W₁.

18. Conjugate comprising a biological substance to which at least one metal complex of the general formula (I) is covalently coupled:
[M(L₁L₂L₃)]ₙ - Xₘ A (I)
in which
- M is a divalent or trivalent metal cation selected from rare earth or transition metal ions,
- L₁, L₂ and L₃ are the same or different and denote ligands with at least two nitrogen-containing heterocycles wherein L₁, L₂ and L₃ are bound to the metal cation via nitrogen atoms,
- X is a reactive or activatable functional group which is covalently bound to at least one of the ligands L₁, L₂ and L₃,
- n is an integer from 1 to 10,
- m is an integer from 1 to 5 and
- A denotes one or several negatively charged groups that are required to balance the charge
wherein the complex contains at least one hydrophilic group selected from C₂-C₃ alkyleneoxy units, C₂-C₃ alkylenethio units, C₂-C₃ alkyleneamino units or/and polyhydroxy units where the hydrophilic groups are components of substituents of the ligands of the metal complex and are present in a linker between one of the ligands and the group X or/and in single substituents which do not contain the group X.

19. Conjugate as claimed in claim 18,
**wherein** the biological substance is biotin, an antibody or antibody fragment, a nucleic acid, a polypeptide antigen, an immunological reactive peptide or a hapten.

20. Use of metal complexes as claimed in one of the claims 1 to 17 or conjugates as claimed in claim 18 or 19 in an immunological detection method.

21. Use as claimed in claim 20 in an electrochemiluminescence method.

22. Use as claimed in claim 21,
**wherein**
the electrochemiluminescence measurement is carried out in the presence of a reducing agent for the metal complex.

23. Use as claimed in claim 22,
**wherein**
the reducing agent is integrated into the ligand sphere of the complex.

## Revendications

1. Complexes métalliques ayant la formule générale (l) :
[M(L₁L₂L₃)]ₙ - XₘA (I)
dans laquelle :
- M est un cation métallique bi ou trivalent choisi à partir d'ions métalliques de transition ou de terres rares,
- L₁, L2, et L₃ sont identiques ou différents et signifient des ligands ayant au moins deux hétérocycles contenant de l'azote, L₁, L₂ et L₃ étant liés par le biais d'atome d'azote ou cations métalliques,
- X est un groupe fonctionnel réactif ou activable qui est lié au moins à l'un des ligands L₁, L₂ et L₃ par covalence,
- n est un nombre entier de 1 à 10,
- m est un nombre entier de 1 à 6 et
- A signifie 1 ou plusieurs groupes chargés négativement nécessaires pour l'équilibrage de la charge,
caractérisé en ce que,
le complexe contient au moins un groupe hydrophile choisi à partir d'unités alkylèneoxy C₂-C₃, unités alkylènethio C₂-C_{3,} unités alkylèneamino C₂-C₃ ou/et unités polyhydroxy, dans la mesure où le groupe X n'est pas un groupe hydroxy, méthoxy-4-méthoxyphénoxy, dioxolane ou CO₂CH₃, les groupes hydrophiles étant des composant de substituants des ligands du complexe métallique et se présentant dans un lieur entre l'un des ligands et le groupe X ou/et se présentent dans des substituants simples ne contenant par le groupe X.

2. Complexe selon la revendication 1, caractérisé en ce que le cation métallique M est un ion de ruthénium, rhénium, osmium, chrome ou iridium.

3. Complexe selon la revendication 1 ou 2, caractérisé en ce que le cation métallique M est un ion de ruthénium.

4. Complexe selon l'une des revendications 1 à 3, caractérisé en ce que les ligands L_{1,} L₂, L₃ contiennent des systèmes cycliques bipyridine ou/et phénanthroline.

5. Complexe selon l'une des revendications 1 à 4, caractérisé en ce que le groupe fonctionnel X réactif ou activable est un groupe halogénure carboxylique, anhydride carboxylique, un ester actif, un maléïde, une amine, un acide caboxylique, un thiol, un halogénure ou un groupe photoactivable.

6. Complexe selon l'une des revendications 1 à 5, caractérisé en ce que les groupes A chargés négativement sont des groupes hexafluorophosphate, trifluoroacétate, tétrafluoroborate ou des ions halogénures.

7. Complexe selon l'une des revendications 1 à 6, caractérisé en ce que les unités polyhydroxy sont choisies à partir des groupes des formules (Ila) et (lIb)
-NR-W (IIa) -O-W (IIb)
W signifiant un radical organique avec au moins deux groupes hydroxy et R signifie l'hydrogène ou alkyle C₁-C₅.

8. Complexe selon la revendication 7, caractérisé en ce que les unités polyhydroxy présentent la formule -NR-C (CH₂OH)₃.

9. Complexe selon l'une des revendications 1 à 8, caractérisé en ce que les unités alkylèneoxy C₂-C₃ alkylènethio C₂-C₃ ou les unités alkylèneamino C₂-C₃ sont des unités éthylèneoxy, éthylènethio ou éthylèneamino.

10. Complexe selon l'une des revendications 1 à 9, caractérisé en ce que 1 jusqu'à 30 unités alkylèneoxy C₂-C₃ alkylènethio C₂-C₃ ou alkylèneamino C₂-C₃ sont présentes par cations métalliques.

11. Complexe selon l'une des revendications 1 à 10, ayant la formule générale (III) : 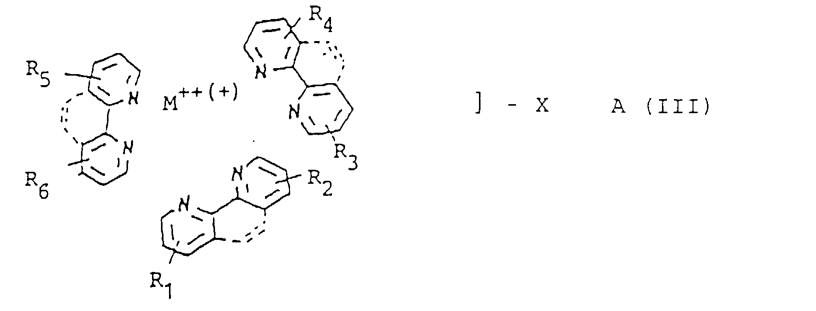 dans laquelle M, X et A sont tels que définis dans la revendication 1, R₁, R₂, R_{3,} R₄, R_{5,} et R₆ sont identiques ou différents et signifient respectivement un ou plusieurs substituants, dans la mesure ou X est lié par l'intermédiaire des substituants R₁, R₂, R₃, R₄, R₅, ou R₆ à l'un des ligands et en ce qu'au moins l'un des substituants R₁, R₂, R₃, R₄, R₅, ou R₆ contient au moins un groupe hydrophile, choisi à partir d'unités alkylèneoxy C₂-C₃ alkylènethio C₂-C₃ et alkylèneamino C₂-C₃.

12. Complexe selon la revendication 11 ayant la formule générale (IIIa) : 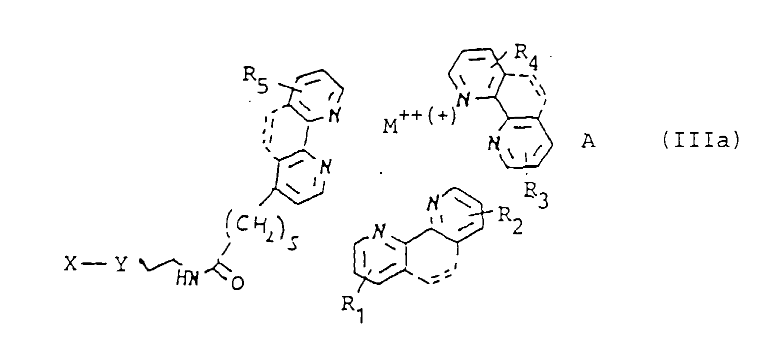 dans laquelle M, X et A sont tels que définis dans la revendication 1, R₁, R₂, R₃, R₄ et R₅ sont tels que définis dans la revendication 11, s est un nombre entier de 0 à 6 et Y est un groupe hydrophile avec une jusqu'à 10 unités hydrophile choisies à partir d'unités alkylèneoxy C₂-C₃, alkylènethio C₂-C₃ et alkylèneamino C₂-C₃.

13. Complexe selon la revendication 1 ayant la formule générale (IIIb) : 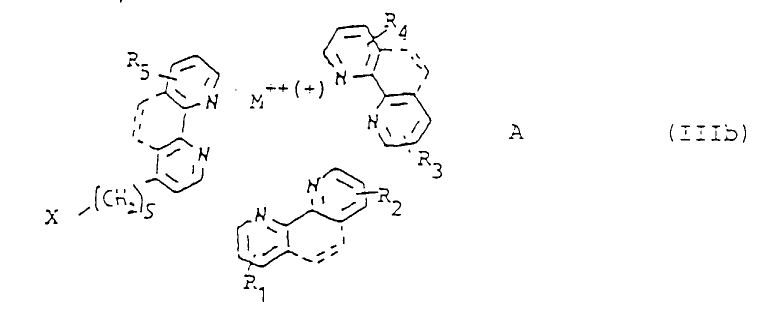 dans laquelle M, X et A sont tels que définis dans la revendication 1, R₁, R₂, R₃, R₄ et R₅ sont tels que définis dans la revendication 11 et s est un nombre entre de 0 à 6 à condition que R₁, R₂, R₃, R₄, R₅ ou/et R₅ contiennent un groupe de substituants hydrophiles qui englobent respectivement 1 jusqu'à 10 unités alkylèneoxy C₂-C₃, alkylènethio C₂-C₃ ou/et alkylèneamino C₂-C₃.

14. Complexe selon l'une des revendications 1 à 10 de formule générale (IV) : 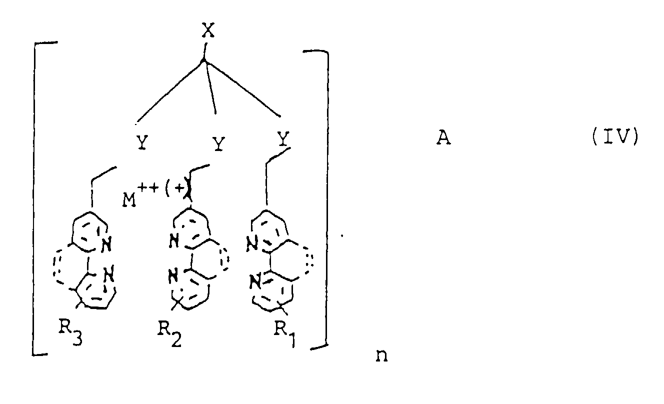 dans laquelle M, X, n et A sont tels que définis dans la revendication 1, R₁, R₂, et R₃ sont identiques ou différents et signifient respectivement un ou plusieurs substituants et Y signifie chaque fois un groupe de lieurs hydrophiles qui contient 1 à 10 unités hydrophiles choisies à partir d'unités alkylèneoxy C₂-C₃, alkylènethio C₂-C₃, et alkylèneamino C₂-C₃.

15. Complexe selon l'une des revendications 1 à 10 de formule générale (V) : 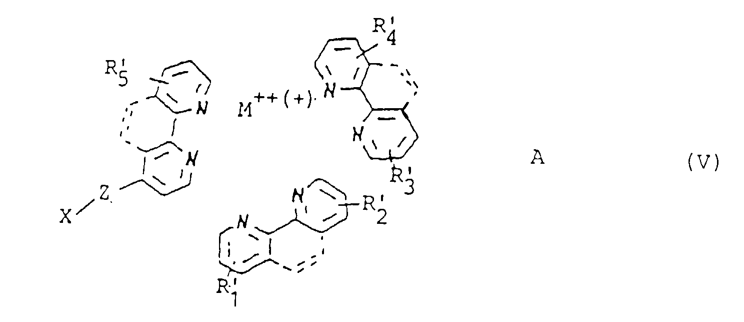 dans laquelle M, X et A sont tels que définis dans la revendication 1, Z signifie un groupe lieur, R'₁, R'₂, R'₃, R'₄ ou/et R'ₛ sont identiques ou différents et signifient chaque fois un ou plusieurs substituants, à condition que R'₁, R'₂, R'₃, ou/et R'₄ contient au moins un groupe de substituants hydrophiles qui contient une unité polyhydroxy.

16. Complexe selon la revendication 15, caractérisé en ce que les groupes OH des unités polyhydroxy sont substitués par d'autres groupes hydrophiles.

17. Complexe selon la revendication 16, caractérisé en ce que les autres groupes hydrophiles englobent un dendrimère ou un élément constitutif dendrimère de la formule (VIA) ou (VIb) :
-A₁-NR-W₁ (A₂-NR-W₂)_{n'} (VIa)
-A₁-O-W₁(A₂-O-W₂)_{n'} (VIb)
dans laquelle
- A₁ et A₂ sont identiques ou différents et signifient un groupe lieur
- W₁ et W₂ sont identiques ou différents et signifient un radical organique avec au moins 2 groupes hydroxy
- R signifie l'hydrogène et un radical alkyle C₁-C₅ et
- n' est égal à 0 et correspond au nombre de groupe hydroxy de W₁

18. Conjugué comprenant une substance biologique à laquelle est accouplé par covalence au moins un complexe métallique ayant la formule générale (I):
[M(L₁L₂L₃)]ₙ - XₘA (I)
dans laquelle
- M est un cation métallique bivalent ou trivalent choisi à partir d'ions des métaux de transition ou de terres rares
- L₁, L₂ et L₃ sont identiques ou différents et signifient des ligands avec au moins deux hétérocycles contenant de l'azote, L₁, L₂ et L₃ étant liés par le biais d'atome d'azote au cation métallique
- X est un groupe fonctionnel réactif ou activable qui est lié par covalence au moins à l'un des ligands L₁, L₂, L₃
- n est un nombre entier de 1 à 10
- m est un nombre entier de 1 à 5
- A signifie un ou plusieurs groupes chargés négativement nécessaires pour la compensation de charge
caractérisé en ce que,
le complexe contient au moins un groupe hydrophile choisi à partir d'unités alkylèneoxy C₂-C₃, d'unités alkylènethio C₂-C₃, d'unités alkylèneamino C₂-C₃ ou/et d'unités polyhydroxy, les groupes hydrophiles sont les composants de substituants des ligands du complexe métallique et se présentent dans un lieur entre l'un des ligands et le groupe X ou/et dans des substituants simples, ne contenant pas le groupe X.

19. Conjugué selon la revendication 18, caractérisé en ce que la substance biologique biotine est un anticorps ou un fragment d'anticorps, un acide nucléique, un antigène polypeptide, un peptide réactif immunologiquement ou un haptène.

20. Utilisation de complexe métallique selon l'une des revendications 1 à 17 conjugués selon la revendication 18 ou 19 dans un procédé de tests immunologiques.

21. Utilisation selon la revendication 20 dans un procédé d'électrochimioluminescence.

22. Utilisation selon la revendication 21, caractérisée en ce que l'on effectue la mesure d'électrochimioluminescence en présence d'un agent réducteur pour le complexe métallique.

23. Utilisation selon la revendication 22, caractérisée en ce que l'agent de réduction est intégré dans la sphère des ligands du complexe.
